(19) Europäisches Patentamt
European Patent Office
Office européen des brevets

(11) **EP 4 552 739 A2**

(12) **EUROPEAN PATENT APPLICATION**

(43) Date of publication:
**14.05.2025 Bulletin 2025/20**

(21) Application number: **25166848.9**

(22) Date of filing: **01.04.2020**

(51) International Patent Classification (IPC):
***B01J 37/08*** (2006.01)

(52) Cooperative Patent Classification (CPC):
**B01J 23/8892; B01J 35/77; B01J 37/086;**
B01J 2235/00; B01J 2235/15; B01J 2235/30

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.04.2019 GB 201904620**

(62) Document number(s) of the earlier application(s) in
accordance with Art. 76 EPC:
**20718735.2 / 3 946 729**

(71) Applicant: **Oxford University Innovation Limited
Oxford, OX2 0JB (GB)**

(72) Inventors:
• **YAO, Benzhen
Oxford OX1 3QR (GB)**

• **EDWARDS, Peter P.
Oxford OX1 3QR (GB)**
• **XIAO, Tiancun
Oxford OX1 3QR (GB)**

(74) Representative: **HGF
HGF Limited
1 City Walk
Leeds LS11 9DX (GB)**

Remarks:
This application was filed on 27-03-2025 as a
divisional application to the application mentioned
under INID code 62.

(54) **IRON - MANGANESE BASED CATALYST, CATALYST PRECURSOR AND CATALYTIC PROCESS**

(57) A catalyst for hydrogenation of carbon dioxide and/or carbon monoxide, wherein the catalyst comprising an iron species, manganese or an oxide thereof, cobalt or an oxide thereof, and an alkali metal, and a process for the hydrogenation of carbon dioxide and/or carbon monoxide using said catalyst to yield hydrocarbons.

**Description**

### *INTRODUCTION*

Described herein is a hydrogenation catalyst, and precursor thereof and its use in a process suitable for the conversion of carbon dioxide and/or carbon monoxide into hydrocarbons. In particular, the catalysts and processes described herein yield $C_{5+}$ hydrocarbons, in particular $C_{5+}$ alpha olefins.

### *BACKGROUND OF THE INVENTION*

Olefins are extensively used in the chemical industry as building blocks for manufacturing a wide range of products and as a main component of fuels. Alpha-olefins have a double bond at the terminal or alpha position which enhances the reactivity of this position and renders them useful in the production of detergents, lubricants, plasticizers, drugs, fine chemicals and polymers.

The catalytic preparation of hydrocarbons from synthesis gas ("syngas") is well known and is commonly referred to as the Fischer-Tropsch synthesis. However, Fischer-Tropsch synthesis tends to favour the formation of saturated hydrocarbon paraffins.

Furthermore, the need to reduce greenhouse gases (GHGs) emissions from the transportation sector is well known. The UK decreased GHG emissions from road transport by 8.6% between 2002-2012 due to increasing fuel efficiency, hydrogen fuel cells and electric vehicles. However, aviation, the second largest transportation subsector, increased its emissions by about 6 %.

Fuel production from $CO_2$ or CO may address the preceding energy demands whilst meeting environmental standards. However, state-of-the-art CO-to-fuel conversion yields mainly $C_1$ products (syngas, formic acid, methanol), and less frequently, $C_2$-$C_4$ products, such as mixed alcohols and olefins. These processes, followed by Methanol-To-Olefin (MTO) or FT synthesis, can yield long-chain hydrocarbon mixtures. However, it is particularly challenging to derive fuel, such as jet fuel, directly via such routes because they fail to produce the desired composition (i.e. comprising $C_{5+}$ hydrocarbons) to meet stringent, well-established standards.

There is a need for new, high performance catalysts and processes suitable for the conversion of carbon dioxide and/or carbon monoxide to hydrocarbons. In particular inexpensive and abundant catalysts are required and processes using said catalysts which improve the conversion of carbon dioxide and/or carbon monoxide, and/or improve the yield of valuable hydrocarbons, such as $C_{5+}$ hydrocarbons, including $C_{5+}$ (alpha) olefins.

### *SUMMARY OF THE INVENTION*

In a first aspect, the present invention relates to a catalyst precursor comprising an iron species, an alkali metal or salt thereof, and a complexing agent.

In a second aspect, the present invention relates to a process for the preparation of a catalyst precursor comprising:

(a) combining (i) an iron species, (ii) an alkali metal or salt thereof, (iii) complexing agent and (iv) a solvent;

(b) agitating the mixture of step (a) to provide a homogenous mixture;

(c) heating the mixture of step (b) to partially remove the solvent and thereby provide a slurry or paste;

In a third aspect, the present invention relates to a catalyst precursor obtainable according to the process of the second aspect.

In a fourth aspect, the present invention relates to a catalyst obtainable by activation of a catalyst precursor according to the first aspect.

In a fifth aspect, the present invention relates to a process for preparing a catalyst comprising:

(a) providing a catalyst precursor according to the first or third aspect of the invention;

(b) optionally subjecting said catalyst precursor to calcination; and

(c) activating said precursor.

In a sixth aspect, the present invention relates to a catalyst obtainable according to the process of the fifth aspect.

In a seventh aspect, the present invention relates to a process for the hydrogenation of carbon dioxide comprising contacting a feedstock comprising hydrogen and carbon dioxide with a catalyst precursor according to the first aspect or a catalyst according to the fourth or sixth aspects at elevated temperature and pressure.

In an eighth aspect, the present invention relates to a process for the hydrogenation of carbon monoxide comprising contacting a feedstock comprising hydrogen and carbon monoxide with a catalyst precursor according to the first aspect or a catalyst according to the fourth or sixth aspect at elevated temperature and pressure.

In a ninth aspect, the present invention relates to a process for the production of olefins comprising contacting a feedstock comprising hydrogen and carbon dioxide and/or carbon monoxide with a catalyst precursor according to the first aspect or a catalyst according to the fourth or sixth aspects at elevated temperature and pressure.

In a tenth aspect, the present invention relates to a process for the production of a fuel comprising contacting a feedstock comprising hydrogen and carbon dioxide and/or carbon monoxide with a catalyst precursor according to the first aspect or a catalyst according to the fourth or sixth aspects at elevated temperature and pressure.

In an eleventh aspect, the present invention relates to a heterogeneous mixture comprising a catalyst precursor according to the first aspect or a catalyst according to the fourth or sixth aspect and a gas comprising hydrogen and carbon monoxide, and/or hydrogen and carbon dioxide.

Preferred, suitable, and optional features of any one particular aspect of the present invention are also preferred, suitable, and optional features of any other aspect.

## BRIEF DESCRIPTION OF THE DRAWINGS

Figure 1 shows a schematic of the equipment used to evaluate catalyst performance.

Figure 2 shows the molar ratio of olefin:paraffin in liquid products produced after $CO_2$ hydrogenation over Fe-Mn-K (100:10:5) [Cat. 3]; Fe-Mn-K (100:10:8) [Cat. 5]; Fe-Mn-K (100:20:5) [Cat. 6].

Figures 3 to 7 show the XRD spectra of various $CO_2$ hydrogenation catalysts.

Figure 8 shows a GC-MS spectrum of the product profile after CO hydrogenation over a Fe-Co-Mn-Na (100:5:20:2) catalyst at 300°C with a syngas feedstock of 1:1 $H_2$:CO.

Figure 9 shows $CO_2$ hydrogenation performance of a Fe-Mn-K catalyst (a): Conversion of $CO_2$ and $H_2$ with reaction time; (b): selectivity of hydrocarbons products with reaction time.

Figure 10 shows GC-MS spectrum of fuel from $CO_2$ hydrogenation over Fe-Mn-K catalyst.

Figure 11 shows XRD spectra of a Fe-Mn-K catalyst precursor, the activated catalyst and the used catalyst.

Figure 12 shows XPS spectra of a Fe-Mn-K catalyst precursor 12a) XPS survey spectra of Fe-Mn-K catalyst; 12b) high resolution XPS spectra of the Fe 2p.

Figure 13 shows SEM images of a) an Fe-Mn-K catalyst precursor and b) the used catalyst.

Figure 14 shows HRTEM images of an Fe-Mn-K catalyst precursor (14 a,b,c) and used catalyst (14 d,e,f).

## DETAILED DESCRIPTION OF THE INVENTION

### Definitions

As used herein the term "catalyst precursor" refers to a material used in the preparation of a catalytically active species. Typically, the precursor is prepared by calcination of the components thereof. Typically, the catalyst precursor will require conversion to the catalytically active species, for instance by oxidation, reduction and/or heat treatment, or a combination thereof. Suitably, activation is via reduction. The catalyst precursor may be converted in the catalytically active species (i.e. "activated") in-situ (i.e. under the reaction conditions) or the catalyst precursor may also be converted to the catalytically active species prior to addition to the reaction.

As used herein the term "liquid" refers to a material which is liquid at standard ambient temperature and pressure (SATP), i.e. at a temperature of 298.15 K (25 °C) and at 100,000 Pa (1 bar, 14.5 psi, 0.9869 atm).

As used herein the term "hydrocarbon" refers to organic compounds consisting of carbon and hydrogen.

For the avoidance of doubt, hydrocarbons include straight-chained and branched, saturated and unsaturated aliphatic hydrocarbon compounds, including alkanes, alkenes, and alkynes, as well as saturated and unsaturated cyclic aliphatic hydrocarbon compounds, including cycloalkanes, cycloalkenes and cycloalkynes, as well as hydrocarbon polymers, for instance polyolefins.

Hydrocarbons also include aromatic hydrocarbons, i.e. hydrocarbons comprising one or more aromatic rings. The aromatic rings may be monocyclic or polycyclic.

Aliphatic hydrocarbons which are substituted with one or more aromatic hydrocarbons, and aromatic hydrocarbons which are substituted with one or more aliphatic hydrocarbons, are also of course encompassed by the term "hydrocarbon" (such compounds consisting only of carbon and hydrogen) as are straight-chained or branched aliphatic hydrocarbons that are substituted with one or more cyclic aliphatic hydrocarbons, and cyclic aliphatic hydrocarbons that are substituted with one or more straight-chained or branched aliphatic hydrocarbons.

A "$C_{n-m}$ hydrocarbon" or "$C_n$-$C_m$ hydrocarbon" or "Cn-Cm hydrocarbon", where n and m are integers, is a hydrocarbon, as defined above, having from n to m carbon atoms. For instance, a $C_{5-16}$ hydrocarbon is a hydrocarbon as defined above which has from 5 to 16 carbon atoms, a $C_{5+}$ hydrocarbon is a hydrocarbon as defined above which has 5 or more carbon atoms etc.

The term "alkane", as used herein, refers to a linear or branched chain saturated hydrocarbon compound. Examples of alkanes, are for instance, butane, pentane, hexane, heptane, octane, nonane, decane, undecane, dodecane, tridecane and tetradecane. Alkanes such as dimethylbutane may be one or more of the possible isomers of this compound. Thus, dimethylbutane includes 2,3-dimethybutane and 2,2-dimethylbutane. This also applies for all hydrocarbon compounds referred to herein including cycloalkane, alkene, cycloalkene.

The term "cycloalkane", as used herein, refers to a saturated cyclic aliphatic hydrocarbon compound. Examples of cycloalkanes include cyclopropane, cyclobutane, cyclopentane, cyclohexane, methylcyclopentane, cycloheptane, methylcyclohexane, dimethylcyclopentane and cyclooctane. Examples of a C5-8 cycloalkane include cyclopentane, cyclohexane, methylcyclopentane, cycloheptane, methylcyclohexane, dimethylcyclopentane and cyclooctane. The terms "cycloalkane" and "naphthene" may be used interchangeably.

The term "alkene", as used herein, refers to a linear or branched chain hydrocarbon compound comprising one or more carbon-carbon double bonds. Examples of alkenes are butene, pentene, hexene, heptene, octene, nonene, decene, undecene, dodecene, tridecene and tetradecene. Alkenes typically comprise one or two double bonds. The terms "alkene" and "olefin" may be used interchangeably. The one or more double bonds may be at any position in the hydrocarbon chain. The alkenes may be cis- or trans-alkenes (or as defined using E- and Z- nomenclature). An alkene comprising a terminal double bond may be referred to as an "alk-1-ene" (e.g. hex-1-ene), a "terminal alkene" (or a "terminal olefin"), or an "alpha-alkene" (or an "alpha-olefin"). The term "alkene", as used herein also often includes cycloalkenes.

The term "cycloalkene", as used herein, refers to partially unsaturated cyclic hydrocarbon compound. Examples of a cycloalkene includes cyclobutene, cyclopentene, cyclohexene, cyclohexa-1,3-diene, methylcyclopentene, cycloheptene, methylcyclohexene, dimethylcyclopentene and cyclooctene. A cycloalkene may comprise one or two double bonds.

The term "aromatic hydrocarbon" or "aromatic hydrocarbon compound", as used herein, refers to a hydrocarbon compound comprising one or more aromatic rings. The aromatic rings may be monocyclic or polycyclic. Typically, an aromatic compound comprises a benzene ring. An aromatic compound may for instance be a C6-14 aromatic compound, a C6-12 aromatic compound or a C6-10 aromatic compound. Examples of C6-14 aromatic compounds are benzene, toluene, xylene, ethylbenzene, methylethylbenzene, diethylbenzene, naphthalene, methyl naphthalene, ethylnaphthalene and anthracene.

As used herein "metal species" is any compound comprising a metal. As such, a metal species includes the elemental metal, metal oxides and other compounds comprising a metal, i.e. metal salts, alloys, hydroxides, carbides, borides, silicides and hydrides. When a specific example of a metal species is stated, said term includes all compounds comprising that metal, e.g. iron species includes elemental iron, iron oxides, iron salts, iron alloys, iron hydroxides, iron carbides, iron borides, iron silicides and iron hydrides for instance.

As used herein, the term "heterogeneous mixture" refers to the physical combination of at least two different substances wherein the two different substances are not in the same phase. For instance, one substance may be a solid and one substance may be a liquid or gas.

**Catalyst precursor**

In one aspect, the present invention relates to a catalyst precursor comprising at least one iron species, an alkali metal or salt thereof and a complexing agent.

In one embodiment, the present invention relates to a catalyst precursor comprising iron or a salt, oxide or hydroxide thereof, an alkali metal or salt thereof, and a complexing agent.

In one embodiment, the complexing agent is suitable for complexing metal cations, in particular iron cations. Accordingly, suitable complexing agents comprise one or more functional groups selected from carboxylic acids, hydroxyl groups, amide groups or amino groups. Suitably, the complexing agent comprises two or more functional groups selected from carboxylic acids, hydroxyl groups, amide groups or amino groups. Suitably the complexing agent is an organic compound.

In one embodiment, the complexing agent or organic compound is selected from a hydroxycarboxylic acid, an aminocarboxylic acid, multicarboxylic acids or salts thereof. Suitably, the complexing agent or organic compound is selected from a hydroxycarboxylic acid and a multicarboxylic acid, or a salt thereof. Alternatively, the complexing agent or organic compound is selected from a hydroxycarboxylic acid and an aminocarboxylic acid, or a salt thereof.

In one embodiment, the complexing agent or organic compound is a bi- or multi-dentate hydroxycarboxylic acid or a salt thereof.

In one embodiment, the complexing agent or organic compound is selected from glycolic acid, lactic acid, hydracylic acid, hydroxybutyric acid, hydroxyvaleric acid, malic acid, mandelic acid, citric acid, sugar acids, tartronic acid, tartaric acid, oxalic acid, malonic acid, maleic acid, tannic acid, succinic acid, salicylic acid, glutaric acid, adipic acid, glycine, hippuric acid, EDTA (ethylenediaminetetraacetic acid), NTA (nitroilotiracetic acid), DTPA (diethylenetriaminepentaacetic acid), HEDTA (N-(2-hydroxyethyl)ethylenediamine-N,N',N'-triacetic acid), alanine, valine, leucine and isoleucine, and salts thereof.

In one embodiment, the complexing agent or organic compound is selected from glycolic acid, lactic acid, hydracylic acid, hydroxybutyric acid, hydroxyvaleric acid, malic acid, mandelic acid, citric acid, sugar acids, tartronic acid, tartaric acid, oxalic acid, malonic acid, maleic acid, tannic acid, succinic acid, salicylic acid, glutaric acid, adipic acid, hippuric acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof.

In one embodiment, the complexing agent or organic compound is selected from hydroxybutyric acid, hydroxyvaleric acid, malic acid, mandelic acid, citric acid, sugar acids, tartronic acid, tartaric acid, oxalic acid, malonic acid, maleic acid, tannic acid, succinic acid, salicylic acid, glutaric acid, adipic acid, hippuric acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof.

In one embodiment, the complexing agent or organic compound is selected from hydroxybutyric acid, hydroxyvaleric acid, malic acid, mandelic acid, citric acid, sugar acids, tartronic acid, tartaric acid, oxalic acid, malonic acid, maleic acid, tannic acid, succinic acid, salicylic acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof.

In one embodiment, the complexing agent or organic compound is selected from citric acid, sugar acids, tartaric acid, oxalic acid, salicylic acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof.

In one embodiment, the complexing agent or organic compound is selected from citric acid, sugar acids, tartaric acid, oxalic acid, salicylic acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof.

In one embodiment, the complexing agent or organic compound is selected from citric acid, tartaric acid, oxalic acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof. Suitably, the complexing agent is citric acid.

In one embodiment, the complexing agent to metal molar ratio is about 0.4:1 to about 4:1. Suitably, the complexing agent to metal molar ratio is about 0.5:1 to about 2:1.

In one embodiment, the complexing agent to metal molar ratio is about 0.8:1 to about 4:1. Suitably, the complexing agent to metal molar ratio is about 1:1 to about 3:1.

In one embodiment, the complexing agent to metal molar ratio is about 0.5:1 to about 5:1. Suitably, the complexing agent to metal molar ratio is about 0.8:1 to about 2:1.

In one embodiment, the complexing agent to iron molar ratio is about 0.5:1 to about 5:1. Suitably, the complexing agent to iron molar ratio is about 0.8:1 to about 2:1.

In one embodiment, the iron species is selected from elemental iron, an iron salt, an iron oxide, an iron alloy, an iron hydroxide, an iron carbide, an iron boride, an iron silicide and an iron hydride. Suitably, the iron species is selected from elemental iron, an iron salt, an iron alloy, an iron hydroxide, and an iron silicide. More suitably, the iron species is selected from elemental iron, an iron salt and an iron hydroxide.

In one embodiment, the iron species is an iron salt. In one embodiment, the iron salt is an iron nitrate, an iron sulphate, an iron halide (suitably iron chloride), or an iron organic acid salt. Suitably, the iron salt is iron (III) nitrate or iron (II) nitrate.

In another embodiment, the iron species is iron powder. The skilled person would understand that iron powder is a commercially available form of elemental iron.

In another embodiment, the iron species is iron oxide, suitably $Fe_3O_4$.

In one embodiment, the catalyst precursor comprises from about 5 to about 90 wt.% Fe. Suitably, the catalyst precursor comprises about 10 to about 90 wt. % of Fe. Suitably, from about 15 to about 90 wt. % of Fe, more suitably from about 20 to about 90 wt. % of Fe, more suitably from about 25 to about 90 wt. % of Fe, more suitably from about 30 to about 90 wt. % of Fe, more suitably from about 40 to about 90 wt. % of Fe, more suitably from about 50 to about 90 wt. % of Fe.

In one embodiment, the catalyst precursor comprises from about 5 to about 80 wt.% Fe. Suitably, the catalyst precursor comprises about 10 to about 80 wt. % of Fe. Suitably, from about 15 to about 80 wt. % of Fe, more suitably from about 20 to about 80 wt. % of Fe, more suitably from about 25 to about 80 wt. % of Fe, more suitably from about 30 to about 80 wt. % of Fe, more suitably from about 40 to about 80 wt. % of Fe, more suitably from about 50 to about 80 wt. % of Fe.

In another embodiment, the catalyst precursor comprises from about 10 to about 90 wt. % Fe. Suitably, the catalyst precursor comprises about 10 to about 80 wt. % of Fe. Suitably, from about 10 to about 70 wt. % of Fe, more suitably from about 10 to about 65 wt. % of Fe.

In another embodiment, the catalyst precursor comprises from about 10 to about 80 wt. % of Fe. Suitably, the catalyst precursor comprises about 10 to about 70 wt. % of Fe. Suitably, from about 10 to about 60 wt. % of Fe, more suitably from about 10 to about 50 wt. % of Fe.

In one embodiment, the alkali metal is selected from potassium, sodium, lithium or caesium. Accordingly, the catalytic precursor may comprise a potassium, sodium, lithium or caesium, or a salt thereof. Suitably, the alkali metal is present as a salt. Suitably, the alkali metal is an alkali metal carbonate, such as potassium carbonate, sodium carbonate, caesium carbonate, lithium carbonate.

In one embodiment, the catalyst precursor comprises from about 0.5 to about 30 wt. % of alkali metal. Suitably, the catalyst precursor comprises about 0.5 to about 25 wt. % of alkali metal. Suitably, from about 0.5 to about 20 wt. % of alkali metal, more suitably from about 0.5 to about 15 wt. % of alkali metal, more suitably from about 0.5 to about 10 wt. % of alkali metal, more suitably from about 0.5 to about 5 wt. % of alkali metal.

In one embodiment, the catalyst precursor comprises from about 1 to about 30 wt. % of alkali metal. Suitably, the catalyst precursor comprises about 1 to about 25 wt. % of alkali metal. Suitably, from about 1 to about 20 wt. % of alkali metal, more suitably from about 1 to about 15 wt. % of alkali metal, more suitably from about 1 to about 10 wt. % of alkali metal, more suitably from about 1 to about 5 wt. % of alkali metal.

In one embodiment, the catalyst precursor may comprise further metal species. Suitably, these further metals will act as promotors in the catalytically active material. In one embodiment, the further metal species is a transition metal species. Suitably, the further metal species is a transitional metal, or salt, oxide or hydroxide thereof.

Suitably, the catalyst precursor further comprises cobalt, chromium, copper, iridium, manganese, molybdenum, palladium, platinum, rhenium, rhodium, ruthenium, strontium, tungsten, vanadium, zinc, or a salt, oxide or hydroxide thereof.

In another embodiment, the catalyst precursor further comprises cobalt, copper, manganese, zinc or a salt, oxide or hydroxide thereof.

In one embodiment, the catalyst precursor comprises manganese oxide.

In one embodiment, the catalyst precursor comprises manganese nitrate.

In one embodiment, the catalyst precursor comprises from about 1 to about 50 wt.% of a further metal species. Suitably, the catalyst precursor comprises about 1 to about 40 wt. % of a further metal species.

In another embodiment, the catalyst precursor comprises from about 5 to about 30 wt. % of a further metal species, more suitably from about 5 to about 20 wt. % of a further metal species, more suitably from about 5 to about 15 wt. % of a further metal species, more suitably from about 5 to about 15 wt. % of a further metal species.

In one embodiment, the catalyst precursor comprises from about 1 to about 30 wt.% of a further metal species. Suitably, the catalyst precursor comprises about 1 to about 25 wt. % of a further metal species. Suitably, from about 1 to about 20 wt. % of a further metal species, more suitably from about 1 to about 15 wt. % of a further metal species, more suitably from about 1 to about 10 wt. % of a further metal species, more suitably from about 1 to about 5 wt. % of a further metal species.

In one embodiment, the catalyst precursor comprises iron or a salt, oxide or hydroxide thereof, at least one further transition metal selected from Mn, Zn, Cu and Co or a salt, oxide or hydroxide thereof, an alkali metal or salt thereof and a complexing agent.

In one embodiment, the catalyst precursor comprises iron or a salt, oxide or hydroxide thereof, at least one further transition metal selected from Mn, Zn, Cu and Co or a salt, oxide or hydroxide thereof, an alkali metal or salt thereof and an organic compound.

In another embodiment, the catalyst precursor comprises iron or a salt, oxide or hydroxide thereof, at least one further transition metal selected from Mn and Co or a salt, oxide or hydroxide, an alkali metal or salt thereof and a complexing agent.

In another embodiment, the catalyst precursor comprises iron or a salt, oxide or hydroxide thereof, at least one further transition metal selected from Mn and Co or a salt, oxide or hydroxide, an alkali metal or salt thereof and an organic compound.

In another embodiment, the catalyst precursor comprises iron or a salt, oxide or hydroxide thereof, a further transition metal selected from Mn and Co or a salt, oxide or hydroxide, an alkali metal or salt thereof and a complexing agent.

In another embodiment, the catalyst precursor comprises iron or a salt, oxide or hydroxide thereof, a further transition metal selected from Mn and Co or a salt, oxide or hydroxide, an alkali metal or salt thereof and an organic compound.

In another embodiment, the catalyst precursor comprises an iron salt, a manganese salt, an alkali metal or salt thereof and a complexing agent.

In another embodiment, the catalyst precursor comprises an iron salt, a manganese salt, an alkali metal or salt thereof and an organic compound.

In another embodiment, the catalyst precursor comprises an iron powder, a manganese salt, a cobalt salt, an alkali metal or salt thereof and a complexing agent.

In another embodiment, the catalyst precursor comprises an iron powder, a manganese salt, a cobalt salt, an alkali metal or salt thereof and an organic compound. In another embodiment, the catalyst precursor comprises an iron nitrate, a manganese nitrate, an alkali metal or salt thereof and a complexing agent.

In another embodiment, the catalyst precursor comprises an iron nitrate, a manganese nitrate, an alkali metal or salt thereof and an organic compound.

Suitably, the alkali metal is potassium. Accordingly, in one embodiment, the catalyst precursor comprises iron or a salt or oxide thereof, at least one further transition metal selected from Mn, Zn, Cu and Co or a salt or oxide thereof, potassium or salt thereof, and a complexing agent.

In another embodiment, the catalyst precursor comprises iron or a salt, oxide or hydroxide thereof, at least one further transition metal selected from Mn and Co or a salt, oxide or hydroxide, potassium or salt thereof and a complexing agent.

In another embodiment, the catalyst precursor comprises iron or a salt, oxide or hydroxide thereof, at least one further transition metal selected from Mn and Co or a salt, oxide or hydroxide, potassium or salt thereof and an organic compound.

Suitably, the complexing agent or organic compound is as defined in one of the afore-mentioned embodiments. Suitably, the cobalt salt is cobalt nitrate. Suitably, the manganese salt is manganese nitrate.

In one embodiment, the catalyst precursor comprises iron (II or III) nitrate, manganese (II) nitrate, potassium carbonate and citric acid. In another embodiment, the catalyst precursor essentially consists of iron (II or III) nitrate, manganese (II) nitrate, potassium carbonate and citric acid.

In another embodiment, the catalyst precursor comprises iron powder, manganese (II) nitrate, cobalt nitrate, sodium carbonate and citric acid. In another embodiment, the catalyst precursor essentially consists of iron powder, manganese (II) nitrate, cobalt nitrate, sodium carbonate and citric acid

In one embodiment, the catalyst precursor comprises (i) Fe or a salt thereof, (ii) Mn or a salt thereof, (iii) K or a salt thereof and (iv) citric acid or a salt thereof.

In one embodiment, the catalyst precursor comprises (i) Fe or a salt thereof, (ii) Mn or a salt thereof, (iii) Co or a salt thereof (iii) K or a salt thereof and (iv) citric acid or a salt thereof.

Suitably, the molar ratio of Fe:Mn is between about 100:1 to about 4:1, more suitably about 15:1 to about 5:1.

Suitably, the molar ratio of Fe:K is about 100:1 to about 2:1; more suitably, the molar ratio of Fe:K is about 20:1 to about 4:1, more suitably about 10:1 to about 2:1.

Suitably, the molar ratio of (Fe+Mn+K):citric acid is between about 5:1 to 0.5:1, suitably about 2:1 to about 1:1.

Suitably, the molar ratio of Fe:Co is about 40:1 to about 10:1, more suitably about 30:1 to about 10:1, more suitably about 20:1.

**Process for preparation of the catalyst precursor**

In a second aspect, the present invention relates to a process for the preparation of a catalyst precursor comprising:

(a) combining (i) at least one iron species, (ii) an alkali metal or salt thereof, (iii) complexing agent and (iv) solvent;

(b) agitating the mixture of step (a) to provide a homogenous mixture;

(c) heating the mixture of step (b) to partially remove the solvent and thereby provide a slurry or paste.

In this aspect, the iron species, alkali metal or salt thereof, and the complexing agent may be as defined in any of the afore-mentioned embodiments.

In one embodiment, the solvent comprises water. Suitably, the solvent is water.

Step (a) may further comprise the addition of one or more further metal species, suitably, a further transition metal species. In one embodiment, a further transition metal selected from Mn, Zn, Co and Cu, or a salt, oxide or hydroxide thereof is combined in step (a).

In step (b) the mixture may be agitated by any means known in the art, such as stirring, shaking, vortexing and sonicating.

In step (c) the mixture is suitably heated to a temperature of from about 30°C to 120°C, more suitably about 30°C to about 80°C, more suitably about 50°C.

The process may further comprise a further step (d) wherein the slurry or paste of step (c) is calcined to provide a powder. Suitably, the calcination is performed at a temperature of between about 300 to about 500°C, more suitably about 350°C. Suitably the calcination is performed in air, suitably static air. Typically, the calcination will result in combustion of organic components of the precursor.

The process may further comprise step (e) wherein the calcined powder is ground or milled, for instance, in order to reduce the particle size.

In another aspect, the present invention relates to a process for the preparation of a catalyst precursor comprising:

(a) combining (i) iron powder (ii) an alkali metal or salt thereof, (iii) complexing agent; and

(b) agitating the mixture of step (a) to provide a homogenous mixture.

In one embodiment, step (a) may further comprise the addition of one or more further metal species. In one embodiment, at least one further transition metal selected from Mn, Zn, Co and Cu, or a salt, oxide or hydroxide thereof is combined in step (a). Suitably, Mn or a salt, oxide or hydroxide thereof and Co or a salt, oxide or hydroxide thereof is further combined in step (a).

In step (b) the agitation may be by any means known in the art, such as stirring, shaking, milling and grinding.

In one embodiment, the process for the preparation of a catalyst precursor comprises:

(a) combining (i) iron powder (ii) potassium, sodium or lithium, or a salt thereof, (iii) citric acid, and (iv) at least one further transition metal selected from Mn and Co, or a salt, oxide or hydroxide thereof; and

(b) agitating the mixture of step (a) to provide a homogenous mixture.

## Catalyst and Process for Preparation thereof

In another aspect, the present invention relates to a catalyst obtainable by activating a catalyst precursor obtainable according to a process described herein, or catalyst obtainable by activating a catalyst precursor described herein.

Suitably, the catalyst is suitable for the hydrogenation of carbon dioxide and/or carbon monoxide.

In one embodiment, the catalyst comprises an iron carbide, suitably $Fe_5C_2$.

In one embodiment, the catalyst comprises an iron carbide, at least one further transition metal selected from Mn, Zn, Cu and Co or a salt, oxide or hydroxide thereof, an alkali metal or salt thereof.

In another embodiment, the catalyst comprises an iron carbide, at least one further transition metal selected from Mn and Co or a salt, oxide or hydroxide, and an alkali metal or salt thereof.

In another embodiment, the catalyst precursor comprises an iron carbide, manganese or oxide thereof, and an alkali metal.

In another embodiment, the catalyst comprises an iron carbide, a manganese or an oxide thereof, cobalt or an oxide thereof, and an alkali metal.

Suitably, the alkali metal is potassium.

In another embodiment, the catalyst comprises an iron carbide, at least one further transition metal selected from Mn and Co or an oxide thereof, and potassium.

Suitably, the iron carbide is $Fe_5C_2$.

Suitably, the molar ratio of Fe:Mn is between about 100:1 to about 4:1, more suitably about 15:1 to about 5:1.

Suitably, the molar ratio of Fe:K is about 100:1 to about 2:1; more suitably, the molar ratio of Fe:K is about 20:1 to about 4:1, more suitably about 10:1 to about 2:1.

Suitably, the molar ratio of Fe:Co is about 40:1 to about 10:1, more suitably about 30:1 to about 10:1, more suitably about 20:1.

In another aspect, the present invention relates to a process for preparing a catalyst comprising:

(a) providing a catalyst precursor as defined in any of the above-mentioned embodiments

(b) optionally subjecting said catalyst precursor to calcination; and

(c) activating said precursor.

In one embodiment, the catalyst is suitable for hydrogenation of carbon dioxide and/or carbon monoxide.

Suitably, the calcination is performed at a temperature of between about 100°C to about 500°C, or about 250°C to about 500°C, more suitably about 300°C to about 350°C. Suitably the calcination is performed in air, suitably static air. Typically, the calcination will result in decomposition or partial combustion of organic components of the precursor.

Step (b) may further comprise grinding or milling the calcined powder in order to reduce the particle size.

The calcined material of step (b) or the precursor of step (a) may be activated, for instance by reduction. Suitably, the material to be activated is exposed to a mixture of CO and hydrogen gas at a temperature of about 250°C to about 500°C, more suitably about 300 to about 350°C.

## Process for Hydrogenation of $CO_2$ or CO

In one aspect, the present invention relates to a process for the hydrogenation of carbon dioxide comprising contacting a feedstock comprising hydrogen and carbon dioxide with a catalyst precursor or a catalyst as defined herein at elevated temperature and pressure.

In another aspect, the present invention relates to a process for the hydrogenation of carbon monoxide comprising contacting a feedstock comprising hydrogen and carbon monoxide with a catalyst precursor or a catalyst as defined herein at elevated temperature and pressure.

In another aspect, the present invention relates to a process for the production of olefins comprising contacting a feedstock comprising (i) hydrogen and (ii) carbon dioxide and/or carbon monoxide, with a catalyst precursor or a catalyst as defined herein at elevated temperature and pressure.

Suitably, the olefins are $C_5$+ olefins, or alpha olefins, or linear olefins. More suitably the olefins are $C_5$+ alpha olefins. Suitably the olefins are liner alpha olefins. More suitably, the olefins are $C_5$+ linear alpha olefins.

Suitably, the olefins are $C_{5-16}$ olefins. More suitably the olefins are $C_{5-16}$ alpha olefins. More suitably, the olefins are $C_{5-16}$ linear alpha olefins.

In another aspect, the present invention relates to a process for the production of hydrocarbons comprising contacting a feedstock comprising (i) hydrogen and (ii) carbon dioxide and/or carbon monoxide, with a catalyst precursor or a catalyst as defined herein at elevated temperature and pressure.

Suitably, the hydrocarbons are $C_5$+ hydrocarbons, more suitably $C_8$-$C_{18}$ hydrocarbons, more suitably $C_8$-$C_{16}$ hydrocarbons. In one embodiment, the hydrocarbons are $C_8$-$C_{18}$ alkanes, more suitably $C_8$-$C_{16}$ alkanes. In one embodiment, the hydrocarbons are jet fuel range hydrocarbons.

In another aspect, the present invention relates to a process for the production of a fuel comprising contacting a feedstock comprising (i) hydrogen and (ii) carbon dioxide and/or carbon monoxide, with a catalyst precursor or a catalyst as defined herein at elevated temperature and pressure.

Suitably, the fuel is selected from gasoline, diesel and aviation/jet fuel.

In conducting carbon monoxide or carbon dioxide hydrogenation or olefin production, the catalyst or catalyst precursor is charged into a reaction zone. The catalyst having been activated ex situ (for instance by heating, or if required by oxidation and subsequent reduction with syngas or hydrogen). The catalyst precursor may be activated in situ, for instance, under the conditions of the reaction.

The catalyst may be used in a fixed bed, a moving bed, ebulating bed, fluidized bed, or slurry bed reactor. Suitably, the catalyst is used in a fixed bed reactor.

In one embodiment, when CO hydrogenation is desired, the feedstock comprising a mixture of hydrogen and carbon monoxide, at suitable $H_2$:CO molar ratio, is contacted with the bed of catalyst, and reacted at reaction conditions. Generally, the molar ratio of $H_2$:CO ranges from about 0.4:1 to about 6:1, suitably from about 0.5:1 to about 3:1, more suitably about 1:1 to about 2:1.

In another embodiment, when $CO_2$ hydrogenation is desired, the feedstock comprising a mixture of hydrogen and carbon dioxide, at suitable $H_2$:$CO_2$ molar ratio, is contacted with the bed of catalyst, and reacted at reaction conditions. Generally, the molar ratio of $H_2$:$CO_2$ ranges from about 0.4:1 to about 8:1, suitably about 0.4:1 to about 6:1, suitably from about 0.5:1 to about 5:1, more suitably about 1:1 to about 4:1. Suitably, the molar ratio of $H_2$:$CO_2$ ranges from about 0.5:1 to about 4:1, more suitably about 1:1 to about 3:1.

The reaction temperatures are elevated. As used herein elevated temperature is a temperature which is elevated with respect to standard ambient temperature, i.e. a temperature of 298.15 K (25 °C). In one embodiment, the feedstock is contacted with the catalyst precursor or catalyst at a temperature of about 180°C to about 500°C, suitably from about 250°C to about 500°C, more suitably about 280°C to about 350°C, or about 300°C to about 350°C.

The reaction pressures are elevated. As used herein elevated pressure is a pressure which is elevated with respect to standard ambient pressure, i.e. a pressure of 100,000 Pa (1 bar, 14.5 psi, 0.9869 atm). In one embodiment, the feedstock is contacted with the catalyst precursor or catalyst at a pressure of about 500KPa to about 10 MPa, suitably about 500KPa to about 5 MPa, suitably about 500KPa to about 2 MPa, suitably about 1 MPa.

The invention will now further be described by means of the following numbered paragraphs:

1. A catalyst precursor comprising an iron species (suitably iron or a salt, oxide or hydroxide thereof) an alkali metal or salt thereof and a complexing agent.

2. A catalyst precursor according to paragraph 1 wherein the complexing agent comprises one or more functional groups selected from carboxylic acids, hydroxyl groups, amide groups or amino groups.

3. A catalyst precursor according to paragraph 1 wherein the complexing agent comprises one or more functional groups selected from carboxylic acids, hydroxyl groups, and amide groups.

4. A catalyst precursor according to paragraph 1 wherein the complexing is selected from a hydroxycarboxylic acid, an aminocarboxylic acid and multicarboxylic acids or salts thereof.

5. A catalyst precursor according paragraph 1 wherein the complexing agent is selected from glycolic acid, lactic acid, hydracylic acid, hydroxybutyric acid, hydroxyvaleric acid, malic acid, mandelic acid, citric acid, sugar acids, tartronic acid, tartaric acid, oxalic acid, malonic acid, maleic acid, tannic acid, succinic acid, salicylic acid glutaric acid, adipic acid, glycine, hippuric acid, EDTA (ethylenediaminetetraacetic acid), NTA (nitroilotiracetic acid), DTPA (diethylenetriaminepentaacetic acid), HEDTA (N-(2-Hydroxyethyl)ethylenediamine-N,N',N'-triacetic acid), alanine, valine, leucine and isoleucine, and salts thereof.

6. A catalyst precursor according paragraph 1 wherein the complexing agent is selected from citric acid, tartaric acid, oxalic acid, EDTA (ethylenediaminetetraacetic acid), NTA (nitroilotiracetic acid), DTPA (diethylenetriaminepentaacetic acid), and HEDTA (N-(2-hydroxyethyl)ethylenediamine-N,N',N'-triacetic acid), or a salt thereof.

7. A catalyst precursor according to any one of the preceding paragraphs wherein the complexing agent is citric acid and/or salt thereof.

8. A catalyst precursor according to any one of the preceding paragraphs wherein the alkali metal is selected from potassium, sodium, lithium and caesium.

9. A catalyst precursor according to any one of the preceding paragraphs wherein the alkali metal is selected from potassium, sodium and caesium.

10. A catalyst precursor according to any one of the preceding paragraphs wherein the alkali metal is potassium.

11. A catalyst precursor according to any one of the preceding paragraphs wherein the iron species is an iron nitrate salt, suitably iron (II) nitrate or iron (III) nitrate.

12. A catalyst precursor according to any one of paragraphs 1 to 10 wherein the iron species is selected from elemental iron, an iron oxide, an iron salt or iron hydroxide, suitably wherein the iron species is iron powder.

13. A catalyst precursor according to any one of the preceding paragraphs comprising a further metal species.

14. A catalyst precursor according to paragraph 13 wherein the further metal species is present at about 1 to about 20 wt. %.

15. A catalyst precursor according to any one of the preceding paragraphs further comprising one or more transition metals selected from Mn, Zn, Co and Cu, or salts, oxides or hydroxides thereof.

16. A catalyst precursor according to paragraph 15 wherein the transition metal is selected from Mn and Co, or salts, oxides and hydroxides thereof.

17. A catalyst precursor according to any one of the preceding paragraphs comprising (i) Fe or a salt thereof, (ii) Mn or a salt thereof, (iii) K or a salt thereof and (iv) citric acid or a salt thereof.

18. A catalyst precursor according to paragraph 17 further comprising (v) Co or a salt thereof.

19. A catalyst precursor according to any one of paragraphs 1 to 11 and 13 to 17 comprising iron (III) nitrate, manganese (II) nitrate, potassium carbonate and citric acid.

20. A catalyst precursor according to any one of paragraphs 1 to 10 and 12 to 18 comprising iron powder, manganese (II) nitrate, cobalt nitrate, sodium carbonate and citric acid.

21. A catalyst precursor according to any one of the preceding paragraphs wherein the molar ratio of Fe:alkali metal is about 100:1 to about 4:1; suitably about 20:1 to about 4:1, more suitably about 10:1.

22. A catalyst precursor according to any one of the preceding paragraphs wherein the complexing agent to Fe molar ratio is about 1:1 to about 3:1.

23. A catalyst precursor according to any one of paragraphs 15 to 20 wherein the molar ratio of Fe:Mn is between about 100:1 to about 4:1, suitably about 10:1.

24. A catalyst precursor according to any one of paragraphs 15 to 20 wherein the molar ratio of (Fe+Mn+K):citric acid is between about 5:1 to 0.5:1, suitably about 2:1 to about 1:1.

25. A catalyst precursor according to any one of paragraphs 15, 16, 18 and 20 wherein the molar ratio of Fe:Co is about 40:1 to about 10:1, more suitably about 30:1 to about 10:1, more suitably about 20:1.

26. A process for the preparation of a catalyst precursor comprising:

(a) combining (i) an iron species (suitably iron or a salt, oxide or hydroxide thereof) (ii) an alkali metal or salt thereof, (iii) complexing agent and (iv) solvent;

(b) agitating the mixture of step (a) to provide a homogenous mixture;

(c) heating the mixture of step (b) to partially remove the solvent and thereby provide a slurry or paste;

27. A process according to paragraph 26 wherein step (a) further comprises combining one or more transition metals selected from Mn, Zn, Co and Cu, or salts, oxides or hydroxides thereof.

28. A process according to any one of paragraphs 26 to 27 wherein step (a) comprises combining (i) Fe or a salt thereof, (ii) Mn or a salt thereof, (iii) K or a salt thereof and (iv) citric acid or a salt thereof.

29. A process according to any one of paragraphs 26 to 28 wherein the iron species in step (a) is iron (III) nitrate.

30. A process according to any one of paragraphs 26 to 29 wherein step (a) comprises combining iron (III) nitrate, manganese (II) nitrate, potassium carbonate and citric acid.

31. A process according to any one of paragraphs 26 to 30 wherein step (a) comprises combining iron (III) nitrate, manganese (II) nitrate, potassium carbonate and citric acid with water.

32. A process according to paragraph 28 wherein the weight ratio of (i) to (iv) in step (a) to solvent is between about 3:1 to about 1:3, suitably about 2:1.

33. A process according to any one of paragraphs 26 to 32 further comprising (d) calcination of the paste or slurry of step (c) to provide a powder.

34. A process according to paragraph 33 wherein the calcination is performed at a temperature of between about 100 to about 500°C, suitably about 250 to about 500°C, suitably about 300 to about 350°C.

35. A process according to any one of paragraphs 33 and 34 wherein the calcination is performed in air or an inert atmosphere, suitably static air.

36. A process according to any one of paragraphs 33 to 35 further comprising (e) grinding the powder of step (d).

37. A process for the preparation of a catalyst precursor comprising:

(a) combining (i) iron powder (ii) an alkali metal or salt thereof, (iii) complexing agent; and

(b) agitating the mixture of step (a) to provide a homogenous mixture.

38. A process according to paragraph 37 wherein step (a) further comprises the addition of one or more further metal species.

39. A process according to any one of paragraph 37 and 38 wherein step (a) further comprises combining Mn or a salt, oxide or hydroxide thereof and Co or a salt, oxide or hydroxide thereof.

40. A process according to claim 37 comprising:

(a) combining (i) iron powder (ii) potassium, sodium or lithium, or a salt thereof, (iii) citric acid, and (iv) at least one further transition metal selected from Mn and Co, or a salt, oxide or hydroxide thereof; and

(b) agitating the mixture of step (a) to provide a homogenous mixture.

41. A process according to any one of paragraphs 27 to 36 and 38 to 40 wherein the molar ratio of Fe:Mn in step (a) is between about 100:1 to about 4:1, suitably about 10:1.

42. A process according to any one of paragraphs 26 to 41 wherein the molar ratio of in step (a) Fe:alkali metal is about 100:1 to about 4:1, suitably about 20:1 to about 4:1.

43. A process according to any one of paragraphs 28, 30 to 32 wherein the molar ratio of in step (a) Fe:K is about 10:1.

44. A process according to paragraph 28 wherein the molar ratio of (Fe+Mn+K):citric acid in step (a) is between about 5:1 to 0.5:1, suitably about 2:1 to about 1:1.

45. A process according to any one of paragraphs 26 to 44 wherein the complexing agent to Fe molar ratio is about 1:1 to about 3:1.

46. A process according to any one of paragraphs 26 to 36 wherein step (c) comprises heating the mixture to between about 30 and 80°C, suitably about 50°C.

47. A process according to any one of paragraphs 26 and 37 wherein the complexing agent is as described in any one of paragraphs 2 to 7.

48. A process according to any one of paragraphs 26 and 37 wherein the alkali metal is as described in any one of paragraphs 8 to 10.

49. A process according to paragraph 40 wherein step (a) comprises combining iron powder, manganese (II) nitrate, cobalt nitrate, sodium carbonate and citric acid.

50. A catalyst obtainable by activation of a catalyst precursor as defined in paragraphs 1 to 25, or a catalyst precursor obtainable by a process according to any one of paragraphs 26 to 49.

51. A process for preparing a catalyst comprising:

(a) providing a catalyst precursor according to any one of paragraphs 1 to 25

(b) optionally subjecting said catalyst precursor to calcination; and

(c) activating said precursor.

52. A process according to paragraph 51 wherein the calcination is performed at a temperature of between about 100°C to about 500°C, about 250°C to about 500°C, suitably about 300°C to about 350°C.

53. A process according to any one of paragraphs 51 and 52 wherein the calcination is performed in air or an inert atmosphere, suitably static air.

54. A process according to any one of paragraphs 51 to 53 wherein step (c) comprises reducing the precursor, suitably by exposure to CO and hydrogen.

55. A process according to any one of paragraphs 51 to 54 further comprising (d) grinding or pelletizing the product of step (c).

56. A catalyst obtainable by a process according to any one of paragraphs 51 to 55.

57. A process for the hydrogenation of carbon dioxide comprising contacting a feedstock comprising hydrogen and carbon dioxide with a catalyst precursor according to paragraphs 1 to 25 or a catalyst according to any one of paragraphs 50 and 56 at elevated temperature and pressure.

58. A process according to paragraph 57 wherein the catalyst precursor is a catalyst precursor according to paragraphs 11, 17 and 19.

59. A process according to any one of paragraphs 57 and 58 wherein the molar ratio of hydrogen and carbon dioxide in the feedstock is 0.4:1 to 6:1, suitably about 1:1 to about 3:1.

60. A process for the hydrogenation of carbon monoxide comprising contacting a feedstock comprising hydrogen and carbon monoxide with a catalyst precursor according to paragraphs 1 to 25 or a catalyst according to any one of paragraphs 50 and 56 at elevated temperature and pressure.

61. A process according to paragraph 60 wherein the catalyst precursor is a catalyst precursor according to paragraphs 12, 15 to 18 and 20.

62. A process according to any one of paragraphs 60 and 61 wherein the molar ratio of hydrogen and carbon monoxide in the feedstock is 0.4:1 to 6:1, suitably about 1:1 to about 2:1.

63. A process for the production of olefins comprising contacting a feedstock comprising hydrogen and carbon monoxide, or hydrogen and carbon dioxide with a catalyst precursor according to paragraphs 1 to 25 or a catalyst according to any one of paragraphs 50 and 56 at elevated temperature and pressure.

64. A process according to paragraph 63 wherein the molar ratio of $H_2:CO_2$ or $H_2:CO$ in the feedstock is 0.4:1 to 6:1.

65. A process according to any one of paragraphs 63 and 64 wherein the olefins are $C_{5+}$ olefins, suitably $C_{5+}$ alpha-olefins.

66. A process according to paragraph 65 wherein the $C_{5+}$ olefins are $C_{5-16}$ olefins or $C_{5-16}$ alpha-olefins.

67. A process according to any one of paragraphs 57 to 66 wherein the feedstock is contacted with the catalyst precursor or catalyst at a temperature of about 100°C to about 500°C, suitably about 250°C to about 500°C, suitably about 300°C to about 350°C.

68. A process according to any one of paragraphs 57 to 67 wherein the feedstock is contacted with the catalyst precursor or catalyst at a pressure of about 500KPa to about 2 MPa, suitably about 1 MPa.

69. A process according to any one of paragraphs 57 to 68 wherein the feedstock is contacted with the catalyst precursor or catalyst at a GHSV (gas hourly space velocity) of about 100 to about 20,000 $h^{-1}$, suitably about 1000 to about 5000 $h^{-1}$.

70. A heterogeneous mixture comprising a catalyst precursor according to any one of paragraphs 1 to 25 or a catalyst according to any one of paragraphs 50 and 56 and a gas comprising hydrogen and carbon monoxide, or hydrogen and carbon dioxide.

The invention will now be further described by the following numbered clauses, which are not claims:

1. A catalyst precursor comprising an iron species, an alkali metal or salt thereof and a complexing agent.

2. A catalyst precursor according to clause 1 wherein the complexing agent comprises one or more functional groups selected from carboxylic acids, hydroxyl groups, amide groups or amino groups.

3. A catalyst precursor according clause 1 wherein the complexing agent is selected from citric acid, tartaric acid, oxalic acid, EDTA (ethylenediaminetetraacetic acid), NTA (nitroilotiracetic acid), DTPA (diethylenetriaminepentaacetic acid), and HEDTA (*N*-(2-hydroxyethyl)ethylenediamine-*N*,*N'*,*N'*-triacetic acid), or a salt thereof.

4. A catalyst precursor according to any one of the preceding clauses wherein the complexing agent is citric acid or a salt thereof.

5. A catalyst precursor according to any one of the preceding clauses wherein the alkali metal is selected from potassium, sodium, lithium and caesium.

6. A catalyst precursor according to any one of the preceding clauses wherein the iron species is an iron nitrate salt, suitably iron (II) nitrate or iron (III) nitrate.

7. A catalyst precursor according to any one of clauses 1 to 6 wherein the iron species is iron powder.

8. A catalyst precursor according to any one of the preceding clauses further comprising one or more transition metals selected from Mn, Zn, Co and Cu, or salts, oxides or hydroxides thereof.

9. A catalyst precursor according to any one of the preceding clauses comprising (i) Fe or a salt thereof, (ii) Mn or a salt thereof, (iii) K or a salt thereof and (iv) citric acid or a salt thereof.

10. A catalyst precursor according to clause 9 further comprising (v) Co or a salt thereof.

11. A catalyst precursor according to any one of clauses 1 to 6, 8 and 9 comprising iron (III) nitrate, manganese (II) nitrate, potassium carbonate and citric acid.

12. A catalyst precursor according to any one of clauses 1 to 5, 7 to 11 comprising iron powder, manganese (II) nitrate, cobalt nitrate, sodium or potassium carbonate and citric acid.

13. A catalyst precursor according to any one of the preceding clauses wherein the molar ratio of Fe:alkali metal is about 20:1 to about 4:1, suitably about 10:1.

14. A catalyst precursor according to any one of clauses 8 to 13 wherein the molar ratio of Fe:Mn is between about 100:1 to about 4:1, suitably about 10:1.

15. A process for the preparation of a catalyst precursor comprising:

(a) combining (i) an iron species, (ii) an alkali metal or salt thereof, (iii) complexing agent and (iv) solvent;

(b) agitating the mixture of step (a) to provide a homogenous mixture;

(c) heating the mixture of step (b) to partially remove the solvent and thereby provide a slurry or paste.

16. A process according to clauses 15 wherein step (a) comprises combining (i) Fe or a salt thereof, (ii) Mn or a salt thereof, (iii) potassium or a salt thereof and (iv) citric acid or a salt thereof.

17. A process for the preparation of a catalyst precursor comprising:

(a) combining (i) iron powder (ii) an alkali metal or salt thereof, (iii) complexing agent; and

(b) agitating the mixture of step (a) to provide a homogenous mixture.

18. A process according to clause 17 wherein step (a) comprises combining (i) iron powder (ii) potassium, sodium or lithium, or a salt thereof, (iii) citric acid, and (iv) at least one further transition metal selected from Mn and Co, or a salt, oxide or hydroxide thereof.

19. A catalyst suitable for hydrogenation of carbon dioxide and/or carbon monoxide obtainable by activation of a catalyst precursor as defined in clauses 1 to 14, or a catalyst precursor obtainable by a process according to any one of clauses 15 to 18.

20. A process for preparing a catalyst comprising:

(a) providing a catalyst precursor according to any one of clauses 1 to 14

(b) optionally subjecting said catalyst precursor to calcination; and

(c) activating said precursor.

21. A process according to clause 20 wherein step (c) comprises reducing the precursor, suitably by exposure to CO and hydrogen.

22. A catalyst suitable for hydrogenation of carbon dioxide and/or carbon monoxide obtainable by a process according to any one of clauses 20 and 21.

23. A process for the hydrogenation of carbon dioxide comprising contacting a feedstock comprising hydrogen and carbon dioxide with a catalyst precursor according to clauses 1 to 14 or a catalyst according to any one of clauses 19 and 22 at elevated temperature and pressure.

24. A process for the hydrogenation of carbon monoxide comprising contacting a feedstock comprising hydrogen and carbon monoxide with a catalyst precursor according to clauses 1 to 14 or a catalyst according to any one of clauses 19 and 22 at elevated temperature and pressure.

25. A process for the production of olefins comprising contacting a feedstock comprising hydrogen and carbon monoxide, or hydrogen and carbon dioxide with a catalyst precursor according to clauses 1 to 14 or a catalyst according to any one of clauses 19 and 22 at elevated temperature and pressure.

## *EXAMPLES*

### 1. CO$_2$ Hydrogenation

All catalyst component materials were obtained from commercial sources as indicated below and used without further modification.

The general method for preparation of the catalyst utilized an organic combustion method. Typically, iron salt and alkali metal salts were mixed with complexing agent in the desired ratios and stirred in water to provide a homogenous aqueous solution. The solution was heated at about 50°C for 1 to 2 hours to obtain a slurry. The slurry is then ignited in a furnace at about 350°C in static air for 4 hours to provide a catalyst precursor.

For instance, preparation of a Fe-Mn-K catalyst comprised mixing citric acid monohydrate with iron (III) Nitrate nonahydrate, manganese(II) nitrate tetrahydrate and potassium carbonate, wherein the molar ratio of citric acid: (Fe+Mn+K) was about 2, and weight ratio of (Fe and Mn and K-precursors + citric acid)/water was about 2:1. The mixture was stirred to form a homogeneous aqueous solution, and heated at 50°C for 1-2 hours to obtain a citric acid based slurry. This paste is ignited at 350°C (furnace temperature) in static air for 4 hours to produce a powder.

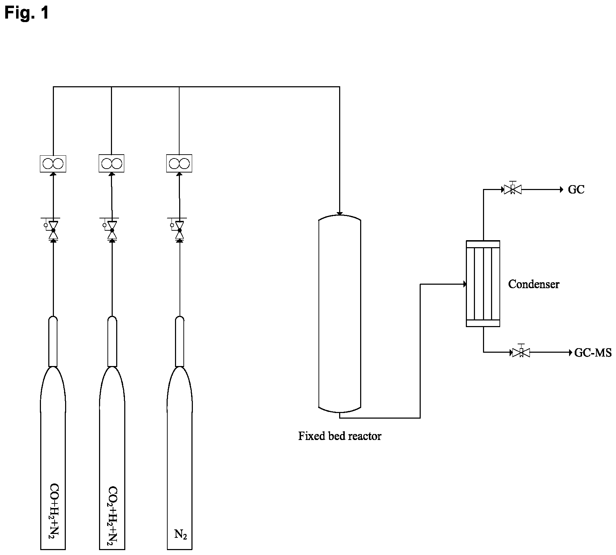

The carbon dioxide hydrogenation experiments are carried on in a fixed bed reactor (Figure 1). Generally, 1.0 g catalyst precursor is mixed with 4.0 g silica carbide and loaded into the reactor. Prior to the reaction, the catalyst precursor is reduced in syngas ($H_2$:CO=2:1) at atmospheric pressure, with a GHSV (gas hourly space velocity) of 1000 mL/g hrs, at 320°C for 24 hours, the heating rate at 2°C/min to provide the activated catalyst.

After reduction, the temperature is decreased to about 50°C, and a mixture of $H_2/CO_2$ (3:1) and $N_2$ (as an internal standard) is used as feedstock gas. Gas flow is set to 40 mL/min (GSVH = 2400 mL/g cat). The $N_2$ was added as inert gas in the syngas feedstock for the conversion calculation. As the mass flow of $N_2$ doesn't change before and after the reaction, the $CO_2$ and $H_2$ conversion, CO and $C_nH_m$ selectivity can be calculated as set out below.

The reactor is heated at a heating rate of 2°C/min until reaction temperature (about 300°C to 320°C). The reaction pressure is controlled at 10 bar (1 Mpa) by a back pressure regulator.

The gaseous products are analyzed on a Perkin Elmer Clarus GC and the collected liquid products are analysed by GC-MS.

The $CO_2$ and $H_2$ conversion, and products selectivity are calculated by the following equations:

$$CO_2 \text{ conversion} = \frac{CO_{2,\text{inlet}} - \frac{N_{2\ inlet}}{N_{2\ outlet}} \times CO_{2,\text{outlet}}}{CO_{2,\text{inlet}}} \times 100\%$$

$$H_2 \text{ conversion} = \frac{H_{2\ inlet} - \frac{N_{2\ inlet}}{N_{2\ outlet}} \times H_{2\ outlet}}{H_{2\ inlet}} \times 100\%$$

$$CO \text{ selectivity} = \frac{\frac{N_{2\ inlet}}{N_{2\ outlet}} \times CO_{\text{outlet}}}{CO_{2,\text{intlet}} - \frac{N_{2\ inlet}}{N_{2\ outlet}} \times CO_{2,\text{outlet}}} \times 100\%$$

$C_n H_m$ selectivity in hydrocarbons (n = 1,2,3,4)

$$= (1 - CO \text{ selectivity}) \times \frac{n \times \frac{N_{2\ inlet}}{N_{2\ outlet}} \times C_n H_{m\ \text{outlet}}}{CO_{2,\text{intlet}} - \frac{N_{2\ inlet}}{N_{2\ outlet}} \times CO_{2,\text{outlet}}} \times 100\%$$

$$C_{5+} \text{ selectivity in hydrocarbons} = (1 - \sum_{n=1}^{4} C_n H_m \text{ selectivity in hydrocarbons}) \times 100\%$$

Table 1 provides examples of Fe-Mn-K catalysts with varying ratios of Fe:Mn:K prepared as set out above with citric acid as the complexing agent. The $H_2$ and $CO_2$ conversion, and products selectivity over the different catalysts after reaction as set out above for a reaction time of 20 hours are shown in Table 1.

## Table 1

| Example | Catalyst | T/°C | Conversion/% | | CO sel. /% | Selectivity hydrocarbons /% | | | | | | | |
|---|---|---|---|---|---|---|---|---|---|---|---|---|---|
| | | | $H_2$ | $CO_2$ | | $CH_4$ | $C_2H_6$ | $C_2H_4$ | $C_3H_8$ | $C_3H_6$ | $C_4H_{10}$ | $C_4H_8$ | $C_{5+}$ |
| 1 | Fe-Mn-K 100:10:5 | 300 | 28.7 | 35.7 | 8.2 | 9.5 | 1.4 | 7.3 | 1.3 | 10.4 | 1.1 | 7.2 | 61.8 |
| 2 | Fe-Mn-K 100:10:8 | 300 | 34.2 | 28.9 | 12.5 | 14.4 | 2.3 | 9.7 | 1.9 | 13.6 | 1.5 | 9.1 | 47.4 |
| 3 | | 320 | 48.2 | 39.3 | 7.6 | 12.1 | 1.9 | 8.2 | 1.8 | 12.2 | 1.4 | 8.2 | 54.2 |
| 4 | Fe-Mn-K 100:20:5 | 300 | 36.8 | 27.6 | 8.5 | 25.0 | 3.9 | 12.6 | 2.3 | 16.9 | 1.6 | 10.4 | 27.2 |
| 5 | | 320 | 38.0 | 29.5 | 10.9 | 16.9 | 2.2 | 10.6 | 1.6 | 14.9 | 1.3 | 10.1 | 42.6 |

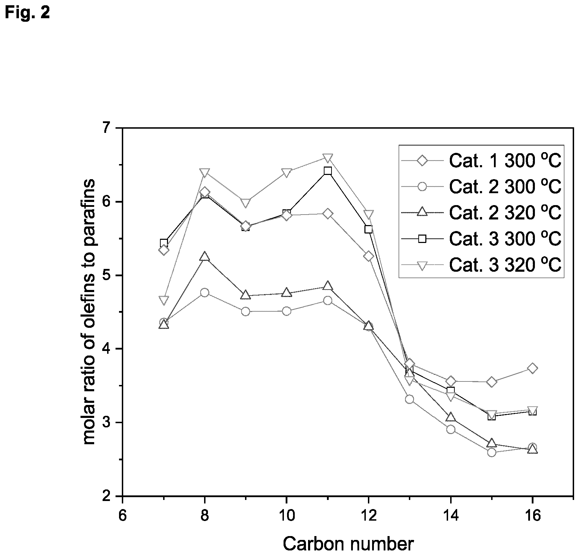

Table 2 and Figure 2 provides the molar ratio of olefins:paraffins for the $C_2$-$C_4$ hydrocarbons produced.

**Table 2**

| Catalyst | T/°C | Molar ratio of olefin:paraffin | | |
|---|---|---|---|---|
| | | C2 | C3 | C4 |
| Fe-Mn-K 100:10:5 | 300 | 5.13 | 7.85 | 6.59 |
| Fe-Mn-K 100:10:8 | 300 | 4.20 | 7.06 | 6.00 |
| | 320 | 4.28 | 6.99 | 5.89 |
| Fe-Mn-K 100:20:5 | 300 | 3.23 | 7.45 | 6.35 |
| | 320 | 4.92 | 9.58 | 8.09 |

Table 2 and Figure 2 show that the catalysts demonstrate higher selectivity for olefins over paraffins in liquid products. The GC-MS spectra for the liquid products showed that the products are concentred at hydrocarbon of $C_6$-$C_{16}$, and the main peaks are assigned to the linear alpha olefins.

The XRD Patterns of each of the catalysts were recorded on Bruker D8 ECO X-ray diffractometer using graphite monochromatized Cu Ka radiation ($\lambda$=0.15418 nm over 2$\theta$ range from 20-80° at a scan rate of 0.02°/s). Most of peaks can be assigned to $Fe_3O_4$.

The crystallites sizes are calculated with the Debye-Scherrer formula based on the peaks of 2$\theta$ = 35.9°:

$$\mathrm{D}_{hkl} = \frac{0.9\,\lambda}{\beta\,cos\theta}$$

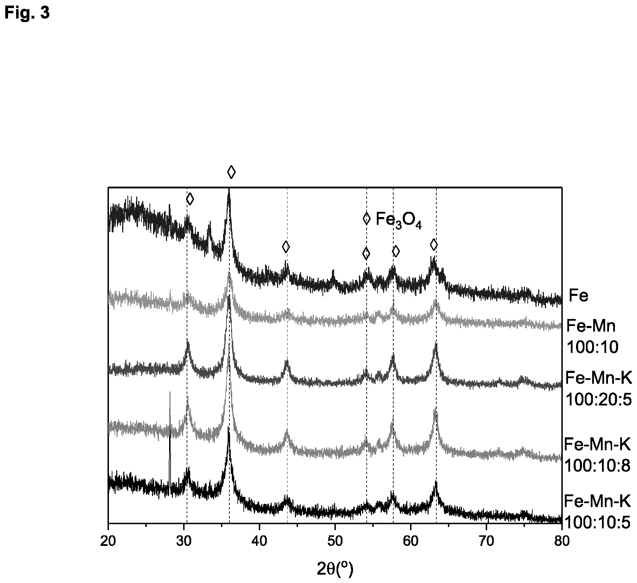

Where $\beta$ is the full-width at half-maximum (FWHM) value of XRD diffraction lines, the wavelength $\lambda$=0.15418 nm and $\theta$ is the half diffraction angle of 2$\theta$. The catalysts showed small crystallites sizes (Table 3), around 10 nm, which are in accordance with the broad peaks in XRD spectrums (Figure 3).

**Table 3**

| Catalyst Molar ratio Fe:Mn:K | 2$\theta$(°) | FWHM (°) | Crystallite size(nm) |
|---|---|---|---|
| 100:0:0 | 35.94 | 0.37 | 13.13 |
| 100:10:0 | 35.93 | 0.30 | 16.42 |
| 100:10:5 | 35.92 | 0.30 | 16.42 |
| 100:10:8 | 35.95 | 0.52 | 9.38 |
| 100:20:5 | 35.98 | 0.67 | 7.30 |

In order to study the effects of various promotors on the $CO_2$ hydrogenation, iron based catalysts were prepared with potassium and various promotors. The catalysts were prepared using an organic combustion method similar to that described above with citric acid as the complexing agent. The catalyst precursors of the catalysts studied in Table 4 were prepared as follows:

Example 4: citric acid monohydrate and iron (III) nitrate nonahydrate molar ratio of 2:1 were dissolved in water to form a homogeneous aqueous solution (weight of (iron(III) nitrate nonahydrate + citric acid monohydrate) to water of about 2:1), and heated at 50°C for 1-2 hours to obtain a citric acid based slurry. This paste is ignited at 350°C (furnace temperature) in static air for 4 hours to produce a powder.

Example 5: citric acid monohydrate, iron (III) nitrate nonahydrate, and potassium carbonate, were dissolved in water to form a homogeneous aqueous solution, wherein the molar ratio of Fe:K of 100:10, and the molar ratio of citric acid: (Fe+K) was about 2, and weight ratio of (iron (III) nitrate nonahydrate + potassium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a powder.

Example 6: citric acid monohydrate, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of citric acid: (Fe+Mn+K) was about 2, and weight ratio of (iron (III) nitrate nonahydrate + manganese(II) nitrate tetrahydrate + potassium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a powder.

Example 7: citric acid monohydrate, iron (III) nitrate nonahydrate, zinc nitrate hexahydrate and potassium carbonate, were dissolved in water to form a homogeneous aqueous solution, wherein the molar ratio of Fe:Zn:K of 100:10:10, the molar ratio of citric acid: (Fe+Zn+K) was about 2, and weight ratio of (iron (III) nitrate nonahydrate + zinc nitrate hexahydrate + potassium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a powder.

Example 8: citric acid monohydrate, iron (III) nitrate nonahydrate, copper(II) nitrate trihydrate and potassium carbonate, were dissolved in water to form a homogeneous aqueous solution, wherein the molar ratio of Fe:Cu:K of 100:10:10, the molar ratio of citric acid: (Fe+Cu+K) was about 2, and weight ratio of (iron (III) nitrate nonahydrate + copper(II) nitrate trihydrate + potassium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a catalyst powder.

Catalyst performance was assessed in $CO_2$ hydrogenation as described above with a reaction time of 20 hours.

Table 4 shows the effect of the inclusion of transition metal (TM) promotors in the catalysts. Catalysts were prepared using citric acid as the complexing agent and had a molar ratio of K:Fe and TM:Fe of 1:10 where applicable. In Table 4, the column titles for the hydrocarbons have the following meanings: $C_{2-4}$=: $C_2$-$C_4$ olefin, $C_{2-4}$0: $C_2$-$C_4$ paraffin; $C_{5+}$: liquid products; $C_{5-16}$=: $C_5$-$C_{16}$ olefin.

## Table 4

| Example | Catalyst | Conversion (%) | | CO sel. (%) | Selectivity in hydrocarbons (%) | | | | | |
|---|---|---|---|---|---|---|---|---|---|---|
| | | $H_2$ | $CO_2$ | | $CH_4$ | $C_{2-4}$= | $C_{2-4}$0 | $C_{5+}$ | $C_{5-16}$= | $C_{5-16}$ alpha olefins |
| 4 | Fe | 43.6 | 37.5 | 5.6 | 39.1 | 10.2 | 23.6 | 27.1 | - | - |
| 5 | Fe-K (100:10) | 39.7 | 41.0 | 5.9 | 10.2 | 23.2 | 3.6 | 63.0 | 46.2 | 43.2 |
| 6 | Fe-Mn-K (100:10:10) | 39.5 | 38.2 | 5.6 | 10.4 | 24.2 | 3.5 | 61.9 | 46.3 | 40.4 |
| 7 | Fe-Zn-K (100:10:10) | 36.1 | 35.5 | 9.1 | 10.5 | 24.3 | 3.6 | 61.7 | 45.0 | 40.0 |
| 8 | Fe-Cu-K (100:10:10) | 39.4 | 38.2 | 8.6 | 8.3 | 18.5 | 4.8 | 68.3 | 46.7 | 42.0 |

Table 5 provides the molar ratio of olefins:paraffins for the $C_2$-$C_4$ hydrocarbons produced.

**Table 5**

| Catalyst | Olefin: paraffin molar ratio | | |
|---|---|---|---|
| | $C_2$ | $C_3$ | $C_4$ |
| Fe | 0.09 | 1.09 | 0.37 |
| Fe-K | 4.39 | 8.56 | 7.29 |
| Fe-Mn-K | 5.17 | 8.47 | 7.17 |
| Fe-Zn-K | 5.23 | 8.18 | 6.96 |
| Fe-Cu-K | 1.76 | 6.41 | 5.45 |

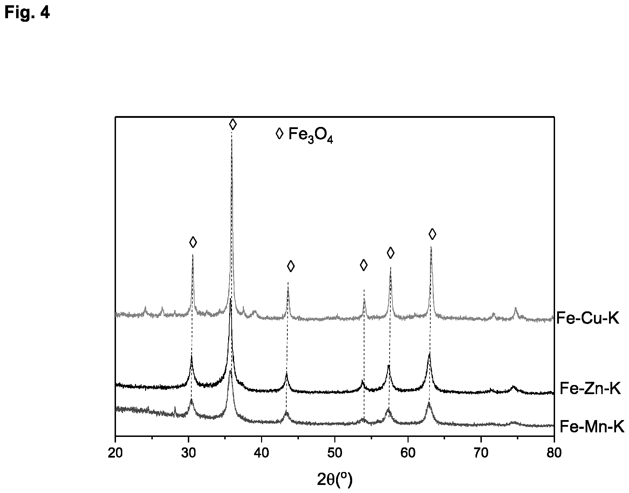

The XRD Patterns (Figure 4) of each of the catalysts were recorded on Bruker D8 ECO X-ray diffractometer using graphite monochromatized Cu Ka radiation ($\lambda$=0.15418 nm over $2\theta$ range from 10-90° at a scan rate of 0.02°/s) and the crystallites sizes are calculated with the Debye-Scherrer formula based as described above. The catalysts showed varying crystallite sizes (Table 6).

**Table 6**

| Catalyst | $2\theta$ | FWHM | d-spacing(Å) | Crystallite size(nm) |
|---|---|---|---|---|
| Fe-Zn-K | 35.73 | 0.13 | 2.51 | 63.84 |
| Fe-Cu-K | 35.91 | 0.11 | 2.50 | 74.52 |
| Fe-Mn-K | 35.75 | 0.60 | 2.51 | 13.97 |

In order to study the effects of various alkali metals on the $CO_2$ hydrogenation, iron based catalysts were prepared with a manganese promotor and the alkali metal varied between Na, K and Cs. The catalyst was prepared using an organic combustion method similar to that described above. The catalyst precursors of the catalysts studied in Table 7 were prepared as follows:

Example 9: citric acid monohydrate, iron (III) nitrate nonahydrate, manganese(II) nitrate tetrahydrate and sodium carbonate, were dissolved in water to form a homogeneous aqueous solution, wherein the molar ratio of Fe:Mn:Na of 100:10:10, the molar ratio of citric acid: (Fe+Mn+Na) was about 2, and weight ratio of (iron (III) nitrate nonahydrate + manganese(II) nitrate tetrahydrate + sodium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a powder.

Example 10: citric acid monohydrate, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of citric acid: (Fe+Mn+K) was about 2, and weight ratio of (iron (III) nitrate nonahydrate + manganese(II) nitrate tetrahydrate + potassium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a catalyst powder.

Example 11: citric acid monohydrate, iron (III) nitrate nonahydrate, manganese(II) nitrate tetrahydrate and caesium carbonate, were dissolved in water to form a homogeneous aqueous solution, wherein the molar ratio of Fe:Mn:Cs of 100:10:10, the molar ratio of citric acid: (Fe+Mn+Cs) was about 2, and weight ratio of (iron (III) nitrate nonahydrate + manganese(II) nitrate tetrahydrate + caesium carbonate + citric acid)/water was about 2:1. The mixture was stirred, and heated at 50°C for 1-2 hours to obtain citric acid based slurry. This paste is ignited at 350°C (furnace temperature) in static air for 4 hours to produce a powder.

Catalyst performance was assessed in $CO_2$ hydrogenation as described above with a reaction time of 20 hours.

Table 7 shows the effect of the inclusion of alkali metals (AM) in the catalysts. Catalysts were prepared using citric acid as the complexing agent and had a molar ratio of AM:Fe and Mn:Fe of 1:10. In Table 7, the column titles for the hydrocarbons have the following meanings: $C_{2-4}$=: $C_2$-$C_4$ olefin, $C_{2-4}$0: $C_2$-$C_4$ paraffin; $C_{5+}$: liquid products; $C_{5-16}$=: $C_5$-$C_{16}$ olefin.

## Table 7

| Example | Catalyst | Conversion (%) | | CO sel. (%) | Selectivity in hydrocarbons (%) | | | | | |
|---|---|---|---|---|---|---|---|---|---|---|
| | | $H_2$ | $CO_2$ | | $CH_4$ | $C_{2-4}^=$ | $C_{2-4}^0$ | $C_{5+}$ | $C_{5-16}^=$ | $C_{5-16}$ alpha olefins |
| 9 | Fe-Mn-Na (100:10:10) | 38.4 | 35.2 | 6.5 | 13.4 | 25.8 | 4.9 | 55.9 | 40.3 | 33.1 |
| 10 | Fe-Mn-K (100:10:10) | 39.5 | 38.2 | 5.6 | 10.4 | 24.2 | 3.5 | 61.9 | 46.3 | 40.4 |
| 11 | Fe-Mn-Cs (100:10:10) | 36.0 | 33.7 | 9.2 | 11.95 | 23.8 | 3.8 | 60.4 | 42.2 | 37.7 |

Table 8 provides the molar ratio of olefins:paraffins for the $C_2$-$C_4$ hydrocarbons produced.

Table 8

| Catalyst | Olefin: paraffin molar ratio | | |
|---|---|---|---|
| | $C_2$ | $C_3$ | $C_4$ |
| Fe-Mn-Na | 0.27 | 2.17 | 1.09 |
| Fe-Mn-K | 2.76 | 8.62 | 7.40 |
| Fe-Mn-Cs | 5.17 | 8.47 | 7.17 |

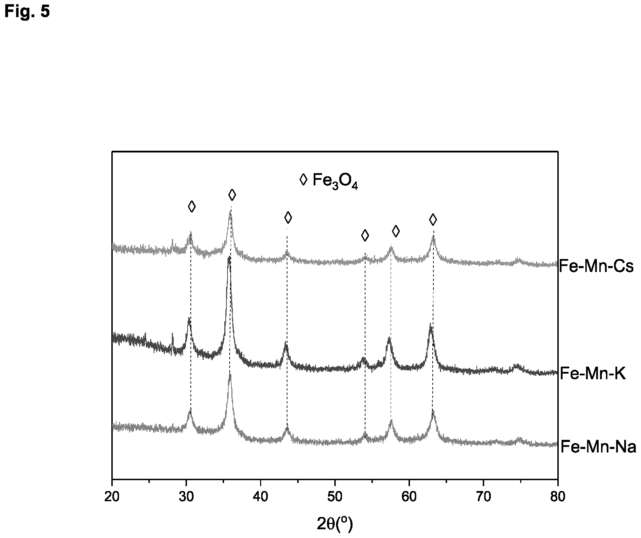

The XRD Patterns (Figure 5) of each of the catalysts were recorded on Bruker D8 ECO X-ray diffractometer using graphite monochromatized Cu Ka radiation ($\lambda$=0.15418 nm over $2\theta$ range from 10-90° at a scan rate of 0.02°/s) and the crystallites sizes are calculated with the Debye-Scherrer formula based as described above. The catalysts showed varying crystallite sizes (Table 9).

**Table 9**

| Catalyst | $2\theta$ | FWHM | d-spacing(Å) | Crystallite size(nm) |
|---|---|---|---|---|
| Fe-Mn-Na | 35.98 | 0.260 | 2.50 | 31.96 |
| Fe-Mn-K | 35.75 | 0.60 | 2.51 | 13.97 |
| Fe-Mn-Cs | 36.03 | 0.30 | 2.49 | 27.96 |

In order to study the effects of the complexing agent used in the preparation of the catalysts on the catalyst performance, a series of iron based catalysts were prepared using a variety of complexing agents. The catalysts included potassium and manganese in a molar ratio to Fe of 1:10. The catalysts were prepared using an organic combustion method similar to that described above. The catalyst precursors of the catalysts studied in Table 10 were prepared as follows:

Example 12 (reference): iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, and weight ratio of (iron (III) nitrate nonahydrate + manganese(II) nitrate tetrahydrate + potassium carbonate)/water was about 2:1. The mixture was stirred, and heated at 50°C for 1-2 hours to obtain a water free mixture. This mixture is calcinated at 350°C (furnace temperature) in static air for 4 hours to produce a powder.

Example 13: urea, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of urea: (Fe+Mn+K) was about 2, and weight ratio of (iron (III) nitrate nonahydrate + manganese(II) nitrate tetrahydrate + potassium carbonate + urea)/water was about 1:1. The mixture was stirred, and heated at 50°C for 1-2 hours to obtain urea based slurry. This paste is ignited at 350°C (furnace temperature) in static air for 4 hours to produce a powder.

Example 14: tannic acid, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of tannic acid: (Fe+Mn+K) was about 2, and weight ratio of (iron (III) nitrate nonahydrate + manganese(II) nitrate tetrahydrate + potassium carbonate + tannic acid)/water was about 1:1. The mixture was stirred, and heated at 50°C for 1-2 hours to obtain tannic acid based slurry. This paste is ignited at 350°C (furnace temperature) in static air for 4 hours to produce a powder.

Example 15: Ethylenediaminetetraacetic acid (EDTA), iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of EDTA: (Fe+Mn+K) was about 2, and weight ratio of (iron (III) nitrate nonahydrate + manganese(II) nitrate tetrahydrate + potassium carbonate + EDTA)/water was about 1:1. The mixture was stirred, and heated at 50°C for 1-2 hours to obtain an EDTA based slurry. This paste is ignited at 350°C (furnace temperature) in static air for 4 hours to produce a powder.

Example 16: citric acid, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of citric acid: (Fe+Mn+K) was about 2, and weight ratio of (iron (III) nitrate nonahydrate + manganese(II) nitrate tetrahydrate + potassium carbonate + citric acid)/water was about 1:1. The mixture was stirred, and heated at 50°C for 1-2 hours to obtain citric acid based slurry. This paste is ignited at 350°C (furnace temperature) in static air for 4 hours to produce a catalyst powder.

Example 17: glycine, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of glycine: (Fe+Mn+K) was about 2, and weight ratio of (iron (III) nitrate nonahydrate + manganese(II) nitrate tetrahydrate + potassium carbonate + glycine)/water was about 1:1. The mixture was stirred, and heated at 50°C for 1-2 hours to obtain glycine based slurry. This paste is ignited at 350°C (furnace temperature) in static air for 4 hours to produce a powder.

Example 18: oxalic acid, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of oxalic acid: (Fe+Mn+K) was about 2, and weight ratio of (iron (III) nitrate nonahydrate + manganese(II) nitrate tetrahydrate + potassium carbonate + oxalic acid)/water was about 1:1. The mixture was stirred, and heated at 50°C for 1-2 hours to obtain oxalic acid based slurry. This paste is ignited at 350°C (furnace temperature) in static air for 4 hours to produce a powder.

Example 19: Nitrilotriacetic acid (NTA), iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of NTA: (Fe+Mn+K) was about 2, and weight ratio of (iron (III) nitrate nonahydrate + manganese(II) nitrate tetrahydrate + potassium carbonate + NTA)/water was about 1:1. The mixture was stirred, and heated at 50°C for 1-2 hours to obtain NTA based slurry. This paste is ignited at 350°C (furnace temperature) in static air for 4 hours to produce a powder.

Example 20: Diethylenetriaminepentaacetic acid (DTPA), iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of DTPA: (Fe+Mn+K) was about 2, and weight ratio of (iron (III) nitrate nonahydrate + manganese(II) nitrate tetrahydrate + potassium carbonate + DTPA)/water was about 1:1. The mixture was stirred, and heated at 50°C for 1-2 hours to obtain DTPA based slurry. This paste is ignited at 350°C (furnace temperature) in static air for 4 hours to produce a powder.

Example 21: tartaric acid, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of tartaric acid: (Fe+Mn+K) was about 2, and weight ratio of (iron (III) nitrate nonahydrate + manganese(II) nitrate tetrahydrate + potassium carbonate + tartaric acid)/water was about 1:1. The mixture was stirred, and heated at 50°C for 1-2 hours to obtain tartaric acid based slurry. This paste is ignited at 350°C (furnace temperature) in static air for 4 hours to produce a powder.

Example 22: Hydroxyethylethylenediaminetriacetic Acid (HEDTA), iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of HEDTA: (Fe+Mn+K) was about 2, and weight ratio of (iron (III) nitrate nonahydrate + manganese(II) nitrate tetrahydrate + potassium carbonate + HEDTA)/water was about 1:1. The mixture was stirred, and heated at 50°C for 1-2 hours to obtain HEDTA based slurry. This paste is ignited at 350°C (furnace temperature) in static air for 4 hours to produce a powder.

Example 23: salicylic acid, iron (III) nitrate nonahydrate, manganese(II) nitrate tetrahydrate and potassium carbonate, were dissolved in water to form a homogeneous aqueous solution, wherein the molar ratio of Fe:Mn:K of 100:10:10, the molar ratio of salicylic acid: (Fe+Mn+K) was about 2, and weight ratio of (iron (III) nitrate nonahydrate + manganese(II) nitrate tetrahydrate + potassium carbonate + salicylic acid)/water was about 1:1. The mixture was stirred, and heated at 50°C for 1-2 hours to obtain salicylic acid based slurry. This paste is ignited at 350°C (furnace temperature) in static air for 4 hours to produce a powder.

Example 24: sugar (commercial granulated sugar), iron (III) nitrate nonahydrate, manganese(II) nitrate tetrahydrate and potassium carbonate, were dissolved in water to form a homogeneous aqueous solution, wherein the weight ratio of Fe:Mn:K of 100:10:10, the molar ratio of sugar: (Fe+Mn+K) was about 2, and weight ratio of (iron (III) nitrate nonahydrate + manganese(II) nitrate tetrahydrate + potassium carbonate + sugar)/water was about 1:1. The mixture was stirred, and heated at 50°C for 1-2 hours to obtain sugar based slurry. This paste is ignited at 350°C (furnace temperature) in static air for 4 hours to produce a powder.

Example 25: flour powder (commercial white wheat flour (plain flour or self-raising flour) ), iron (III) nitrate nonahydrate, manganese(II) nitrate tetrahydrate and potassium carbonate, were dissolved in water to form a homogeneous aqueous slurry, wherein the weight ratio of Fe:Mn:K of 100:10:10, the molar ratio of flour: (Fe+Mn+K) was about 2, and weight ratio of (iron (III) nitrate nonahydrate + manganese(II) nitrate tetrahydrate + potassium carbonate + flour powder)/water was about 1:1. The mixture was stirred, and heated at 50°C for 1-2 hours to obtain flour based slurry. This paste is ignited at 350°C (furnace temperature) in static air for 4 hours to produce a powder.

Catalyst performance was assessed in $CO_2$ hydrogenation as described above with a reaction time of 20 hours.

Table 10 shows that effect of the complexing agent used in the preparation of the catalysts on performance. In Table 10, the column titles for the hydrocarbons have the following meanings: $C_{2-4}$=: $C_2$-$C_4$ olefin, $C_{2-4}$0: $C_2$-$C_4$ paraffin; $C_{5+}$: liquid products; $C_{5-16}$=: $C_5$-$C_{16}$ olefin.

## Table 10

| Example | Catalyst (Fe-Mn-K, 100:10:10) Prepared with complexing agent | Conversion (%) | | CO sel. (%) | Selectivity in hydrocarbons (%) | | | | | |
|---|---|---|---|---|---|---|---|---|---|---|
| | | $H_2$ | $CO_2$ | | $CH_4$ | $C_{2-4}$= | $C_{2-4}$0 | $C_{5+}$ | $C_{5-16}$= | $C_{5-16}$ alpha olefins |
| 12 | none | 27.0 | 28.6 | 6.5 | 14.1 | 26.0 | 5.17 | 54.7 | - | - |
| 13 | Urea | 34.4 | 35.0 | 5.8 | 14.3 | 27.6 | 4.6 | 53.5 | 31.7 | 29.1 |
| 14 | Tannic acid | 39.2 | 38.8 | 5.0 | 16.3 | 26.4 | 4.8 | 52.6 | 26.2 | 24.7 |
| 15 | EDTA | 39.6 | 40.6 | 7.0 | 13.5 | 21.6 | 4.5 | 60.5 | 42.5 | 38.3 |
| 16 | Citric acid | 39.5 | 38.2 | 5.6 | 10.4 | 24.2 | 3.5 | 61.9 | 46.3 | 40.4 |
| 17 | Glycine | 38.7 | 37.4 | 7.6 | 23.7 | 25.9 | 8.5 | 41.9 | 26.3 | 24.6 |
| 18 | Oxalic acid | 36.7 | 37.0 | 7.4 | 9.8 | 25.4 | 3.5 | 61.3 | 45.3 | 40.0 |

| Example | Catalyst (Fe-Mn-K, 100:10:10) Prepared with complexing agent | Conversion (%) | | CO sel. (%) | Selectivity in hydrocarbons (%) | | | | | |
|---|---|---|---|---|---|---|---|---|---|---|
| | | $H_2$ | $CO_2$ | | $CH_4$ | $C_{2-4}$= | $C_{2-4}$0 | $C_{5+}$ | $C_{5-16}$= | $C_{5-16}$ alpha olefins |
| 19 | NTA | 39.5 | 42.5 | 5.1 | 7.2 | 18.3 | 2.6 | 71.9 | 45.4 | 39.6 |
| 20 | DTPA | 42.0 | 44.0 | 6.2 | 9.6 | 19.0 | 3.4 | 68.0 | 43.6 | 41.0 |
| 21 | Tartaric acid | 40.1 | 41.8 | 4.6 | 7.9 | 18.9 | 2.7 | 70.4 | 49.9 | 44.3 |
| 22 | HEDTA | 42.3 | 41.5 | 4.9 | 9.6 | 20.2 | 3.1 | 67.0 | 44.9 | 40.8 |
| 23 | Salicylic acid | 37.8 | 37.3 | 7.2 | 12.6 | 22.1 | 3.8 | 61.5 | 38.4 | 35.4 |
| 24 | Sugar | 36.5 | 37.4 | 8.8 | 10.3 | 21.5 | 3.9 | 64.3 | 40.4 | 36.2 |
| 25 | Flour | 36.5 | 35.6 | 7.2 | 12.1 | 25.9 | 4.0 | 58.0 | 35.4 | 33.6 |

Table 11 provides the molar ratio of olefins: paraffins for the $C_2$-$C_4$ hydrocarbons produced.

**Table 11**

| Catalyst (Fe-Mn-K, 100:10:10) Prepared with | Olefin: paraffin molar ratio | | |
|---|---|---|---|
| | $C_2$ | $C_3$ | $C_4$ |
| none | 3.70 | 6.50 | 5.50 |
| Urea | 4.24 | 7.77 | 6.63 |
| Tannic acid | 3.81 | 7.53 | 6.45 |
| EDTA | 2.99 | 7.12 | 6.19 |
| Citric acid | 5.17 | 8.47 | 7.17 |
| Glycine | 1.82 | 4.76 | 3.84 |
| Oxalic acid | 5.19 | 9.27 | 7.78 |
| NTA | 5.56 | 8.33 | 7.04 |
| DTPA | 4.10 | 7.11 | 6.17 |
| Tartaric acid | 5.33 | 8.49 | 7.24 |
| HEDTA | 4.88 | 8.01 | 6.85 |
| Salicylic acid | 4.12 | 7.62 | 6.53 |
| Sugar | 4.15 | 6.93 | 5.87 |
| Flour powder | 4.70 | 8.47 | 7.11 |

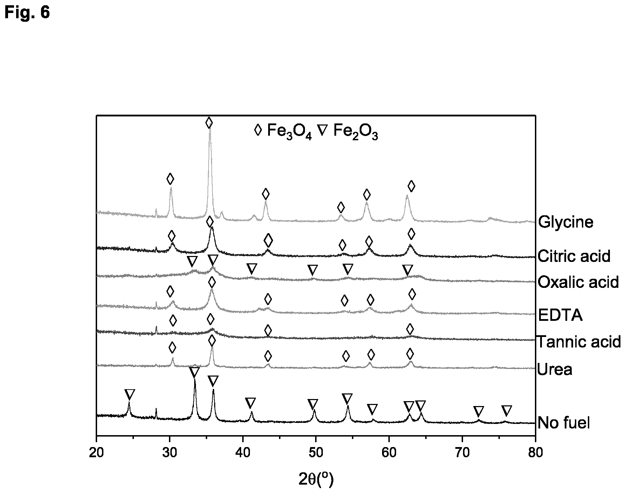

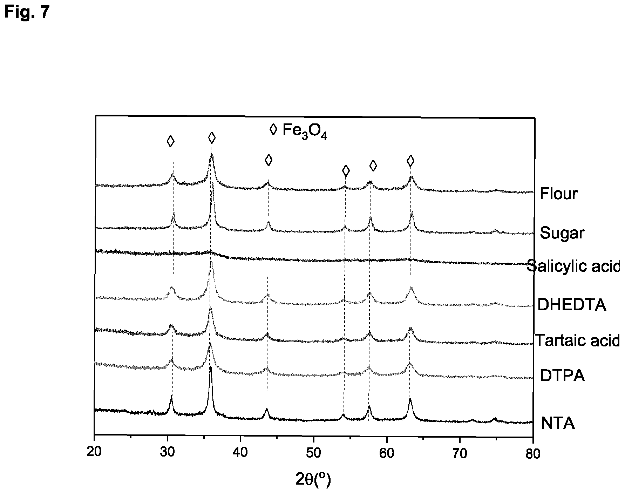

The XRD Patterns (Figures 6 and 7) of each of the catalysts were recorded on Bruker D8 ECO X-ray diffractometer using graphite monochromatized Cu Ka radiation ($\lambda$=0.15418 nm over $2\theta$ range from 10-90° at a scan rate of 0.02°/s) and the crystallites sizes are calculated with the Debye-Scherrer formula based as described above. The catalysts showed varying crystallite sizes (Table 12).

**Table 12**

| Catalyst (Fe-Mn-K, 100:10:10) Prepared with | $2\theta$ | FWHM | d-spacing(Å) | Crystallite size(nm) |
|---|---|---|---|---|
| - | 33.42 | 0.13 | 2.68 | 63.44 |
| Urea | 35.83 | 0.15 | 2.51 | 55.88 |
| Tannic acid | 35.85 | 0.60 | 2.51 | 13.97 |
| EDTA | 35.74 | 0.67 | 2.51 | 12.42 |
| Glycine | 35.46 | 0.30 | 2.53 | 27.92 |
| Citric acid | 35.75 | 0.60 | 2.51 | 13.97 |
| Oxalic acid | 35.87 | 0.45 | 2.50 | 18.63 |
| NTA | 35.94 | 0.34 | 2.50 | 24.85 |
| DTPA | 35.96 | 0.67 | 2.50 | 12.42 |
| Tartaric acid | 35.78 | 0.30 | 2.51 | 27.94 |
| HEDTA | 35.95 | 0.37 | 2.50 | 22.36 |
| Salicylic acid | 35.69 | 1.20 | 2.52 | 6.98 |
| Sugar | 36.04 | 0.11 | 2.49 | 74.55 |
| Flour powder | 35.92 | 0.67 | 2.50 | 12.42 |

The iron based catalysts prepared with complexing agent and with the addition of Na, K and/or Cs improved selectivity of olefin production in the $CO_2$ hydrogenation reaction. The further addition of Mn, Zn and/or Cu promotors also showed high selectivity for olefins over paraffins. Different organic compounds were applied as the complexing agent during the catalyst preparation. The catalysts prepared with citric acid, EDTA, oxalic acid, NTA, DTPA, Tartaric acid, HEDTA showed highest selectivity for olefins. The catalysts can also be applied for the production of fuels (gasoline, diesel, aviation fuel/jet fuel) via $CO_2$ and/or CO hydrogenation.

## 2. CO Hydrogenation

All catalyst component materials were obtained from commercial sources as indicated below and used without further modification.

Typically, catalysts were prepared using iron powder as the iron source. Iron powder, cobalt nitrate, manganese nitrate, alkali metal salts (e.g. potassium, sodium carbonate, lithium carbonate, caesium carbonate) were mixed together, and the mixture was ground to uniformity, the complexing agent (citric acid) was added to the mixture (suitably in about a 1:1 weight ratio with iron), and the mixture ground once more to uniformity. The obtained mixtures were dried at 80°C for 24 hours. The dried mixtures (without calcination) were ground into powder to provide a catalyst precursor.

Prior to the reaction, the catalyst precursor was reduced in syngas ($H_2$:CO=2:1 or 1:1) at atmospheric pressure, with a GHSV (gas hourly space velocity) of 1000 mL/g hrs, at 320°C for 32 hours, the heating rate at 5°C/min to provide the activated catalyst.

After reduction, the temperature is decreased to less than 50°C, and a mixture of $H_2$/CO (1:1) and $N_2$ (as an internal standard) is used as feedstock gas. Gas flow is set to 40 mL/min (GSVH =2 400 mL/g cat). The $N_2$ was added as inert gas in the syngas feedstock for the conversion calculation. As the mass flow of $N_2$ doesn't change before and after the reaction, the CO and $H_2$ conversion, $CO_2$ and $C_nH_m$ selectivity can be calculated as set out below.

The reactor (Figure 1) is heated as a heating rate of 2°C/min until reaction temperature (about 280°C to 320°C). The reaction pressure is controlled at 10 bar (1 Mpa) by a back pressure regulator.

The gaseous products are analyzed on a Perkin Elmer Clarus GC and the collected liquid products are analysed by GC-MS.

The CO and $H_2$ conversion, and products selectivity are calculated by the following equations:

$$\text{CO conversion} = \frac{CO_{\text{inlet}} - \frac{N_{2\ inlet}}{N_{2\ outlet}} \times \text{CO}_{\text{outlet}}}{\text{CO}_{\text{inlet}}} \times 100\%$$

$$H_2 \text{ conversion} = \frac{H_{2\ inlet} - \frac{N_{2\ inlet}}{N_{2\ outlet}} \times H_{2\ outlet}}{H_{2\ inlet}} \times 100\%$$

$C_nH_m$ selectivity in hydrocarbons

$$= (1 - \text{CO}_2 \text{ selectivity}) \times \frac{n \times \frac{N_{2\ inlet}}{N_{2\ outlet}} \times C_nH_{m\ \text{outlet}}}{\text{CO}_{\text{intlet}} - \frac{N_{2\ inlet}}{N_{2\ outlet}} \times \text{CO}_{\text{outlet}}} \times 100\%$$

$$C_{5+} \text{ selectivity in hydrocarbons} = (1 - \sum_{n=1}^{4} C_nH_m \text{ selectivity in hydrocarbons}) \times 100\%$$

Table 13 studies the effect of adding a further transition metal, cobalt, to Fe-Mn-Na catalysts. The reaction time, $H_2$ and CO conversion, and product selectivity over the different catalysts after reaction as set out above are shown in Table 13.

The catalyst precursors of the catalysts studied in Table 13 were prepared as follows:

Examples 26-30: Iron powder, manganese (II) nitrate tetrahydrate, sodium carbonate were mixed together with molar ratio of Fe:Mn:Na of 100:10:2, and the mixture was ground to uniformity, citric acid was added to the mixture and the mixture ground once more to uniformity, wherein the weight ratio of citric acid to iron powder of 4:1. The obtained mixtures were dried at 80°C for 24 hours. The dried mixtures (without calcination) were ground into powder.

Examples 31-35: Iron powder, cobalt (II) nitrate hexahydrate, manganese (II) nitrate tetrahydrate, sodium carbonate were mixed together with molar ratio of Fe:Co:Mn:Na of 10:2:10:2, and the mixture was ground to uniformity, citric acid was added to the mixture and the mixture ground once more to uniformity, wherein the weight ratio of citric acid to iron powder of 4:1. The obtained mixtures were dried at 80°C for 24 hours. The dried mixtures (without calcination) were ground into powder.

Examples 36-44: Iron powder, cobalt (II) nitrate hexahydrate, manganese (II) nitrate tetrahydrate, sodium carbonate were mixed together with molar ratio of Fe:Co:Mn:Na of 100:5:10:2, and the mixture was ground to uniformity, citric acid was added to the mixture and the mixture ground once more to uniformity, wherein the weight ratio of citric acid to iron powder of 4:1. The obtained mixtures were dried at 80°C for 24 hours. The dried mixtures (without calcination) were ground into powder.

Examples 45-50: Iron powder, cobalt (II) nitrate hexahydrate, manganese (II) nitrate tetrahydrate, sodium carbonate were mixed together with molar ratio of Fe:Co:Mn:Na of 100:8:10:2, and the mixture was ground to uniformity, citric acid was added to the mixture and the mixture ground once more to uniformity, wherein the weight ratio of citric acid to iron powder of 4:1. The obtained mixtures were dried at 80°C for 24 hours. The dried mixtures (without calcination) were ground into powder.

Examples 51-55: Iron powder, cobalt (II) nitrate hexahydrate, manganese (II) nitrate tetrahydrate, sodium carbonate were mixed together with molar ratio of Fe:Co:Mn:Na of 100:10:10:2, and the mixture was ground to uniformity, citric acid was added to the mixture and the mixture ground once more to uniformity, wherein the weight ratio of citric acid to iron powder of 4:1. The obtained mixtures were dried at 80°C for 24 hours. The dried mixtures (without calcination) were ground into powder.

## Table 13

| Example | Catalyst | T/°C | Time (hours) | Conversion % | | CO₂ Sel. % | Selectivity in hydrocarbons % | | | | |
|---|---|---|---|---|---|---|---|---|---|---|---|
| | | | | $H_2$ | CO | | $CH_4$ | $C_2\text{-}C_4^=$ | $C_2\text{-}C_4^0$ | $C_5^+ + C_{2\text{-}4}^=$ | $C_5^+$ |
| 26 | Fe-Mn-Na 100:10:2 | 260 | 10 | 3.4 | 3.4 | 62.1 | 22.3 | 33.1 | 6.1 | 71.6 | 38.6 |
| 27 | | 280 | 10 | 22.9 | 24.9 | 42.2 | 10.2 | 25.3 | 4.0 | 85.9 | 60.6 |
| 28 | | 300 | 10 | 19.3 | 27.3 | 50.0 | 16.7 | 26.1 | 7.0 | 76.4 | 50.2 |
| 29 | | 320 | 10 | 15.1 | 23.5 | 52.0 | 22.7 | 27.8 | 5.4 | 71.9 | 44.2 |
| 30 | | 320 | 20 | 11.9 | 18.8 | 53.9 | 24.1 | 29.1 | 4.7 | 71.2 | 42.1 |
| 31 | Fe-Co-Mn-Na 100:2:10:2 | 260 | 10 | 14.3 | 13.9 | 39.4 | 6.8 | 13.5 | 2.4 | 90.8 | 77.3 |
| 32 | | 280 | 10 | 34.5 | 48.3 | 45.9 | 9.4 | 21.9 | 4.1 | 86.5 | 64.7 |
| 33 | | 300 | 10 | 40.3 | 61.6 | 48.6 | 13.3 | 23.5 | 5.4 | 81.3 | 57.8 |
| 34 | | 320 | 10 | 33.2 | 51.7 | 49.4 | 19.0 | 25.2 | 4.6 | 76.5 | 51.3 |
| 35 | | 320 | 19 | 26.1 | 41.6 | 49.6 | 19.4 | 24.7 | 3.9 | 76.7 | 52.0 |
| 36 | Fe-Co-Mn-Na 100:5:10:2 | 260 | 10 | 5.0 | 5.6 | 39.2 | 10.9 | 15.9 | 3.2 | 86.0 | 70.1 |
| 37 | | 280 | 10 | 33.6 | 35.6 | 39.9 | 8.2 | 18.3 | 3.4 | 88.5 | 70.2 |
| 38 | | 300 | 10 | 39.3 | 57.0 | 47.0 | 11.4 | 21.2 | 4.5 | 84.2 | 62.9 |
| 39 | | 320 | 10 | 43.7 | 64.3 | 48.2 | 16.2 | 24.3 | 5.1 | 78.7 | 54.5 |
| 40 | | 320 | 20 | 40.3 | 58.6 | 48.2 | 16.6 | 24.3 | 4.8 | 78.7 | 54.4 |
| 41 | | 320 | 30 | 40.0 | 59.4 | 48.3 | 16.9 | 24.3 | 4.8 | 78.3 | 54.0 |
| 42 | | 320 | 40 | 41.0 | 60.6 | 48.1 | 17.0 | 24.2 | 4.7 | 78.3 | 54.1 |
| 43 | | 320 | 47 | 42.5 | 62.9 | 48.0 | 17.1 | 24.2 | 4.8 | 78.1 | 54.0 |
| 44 | | 320 | 48 | 43.0 | 63.1 | 48.2 | 17.2 | 24.3 | 4.9 | 78.0 | 53.7 |
| 45 | Fe-Co-Mn-Na 100:8:10:2 | 260 | 10 | 14.5 | 12.9 | 29.6 | 8.5 | 15.9 | 3.1 | 88.4 | 72.4 |
| 46 | | 280 | 10 | 21.6 | 26.4 | 43.7 | 9.4 | 20.8 | 2.9 | 87.7 | 66.9 |
| 47 | | 300 | 10 | 24.1 | 37.8 | 49.3 | 13.5 | 23.2 | 3.6 | 82.9 | 59.8 |
| 48 | | 320 | 10 | 25.4 | 40.2 | 49.6 | 20.5 | 24.9 | 3.9 | 75.7 | 50.8 |
| 49 | | 340 | 10 | 39.4 | 62.4 | 49.1 | 24.4 | 24.8 | 3.9 | 71.7 | 46.9 |
| 50 | | 360 | 10 | 75.4 | 96.8 | 44.2 | 27.8 | 12.6 | 14.0 | 58.2 | 45.6 |
| 51 | Fe-Co-Mn-Na 100:10:10:2 | 260 | 10 | 34.3 | 31.8 | 34.2 | 6.8 | 16.0 | 3.1 | 90.1 | 74.1 |
| 52 | | 280 | 10 | 43.22 | 59.2 | 46.5 | 12.1 | 20.9 | 6.7 | 81.2 | 60.4 |
| 53 | | 300 | 10 | 28.7 | 43.5 | 49.5 | 15.1 | 25.1 | 5.2 | 79.8 | 54.7 |

| Example | Catalyst | T/ °C | Time(hours) | Conversion % | | CO$_2$ Sel. % | Selectivity in hydrocarbons % | | | | |
|---|---|---|---|---|---|---|---|---|---|---|---|
| | | | | H$_2$ | CO | | CH$_4$ | C$_2$-C$_4^=$ | C$_2$-C$_4^0$ | C$_5^+$+C$_{2\text{-}4}^=$ | C$_5^+$ |
| 54 | | 320 | 10 | 23.7 | 36.2 | 50.3 | 23.0 | 25.7 | 3.8 | 73.3 | 47.6 |
| 55 | | 320 | 20 | 21.7 | 32.7 | 50.1 | 24.0 | 25.2 | 3.5 | 72.4 | 47.2 |

Table 14 provides the molar ratio of olefins: paraffins for the C$_2$-C$_4$ hydrocarbons produced.

## Table 14

| Example | Catalyst | T/ °C | Time(hours) | Olefin: paraffin molar ratio | | |
|---|---|---|---|---|---|---|
| | | | | C2 | C3 | C4 |
| 26 | Fe-Mn-Na 100:10:2 | 260 | 10 | 4.7 | 6.6 | 5.2 |
| 27 | | 280 | 10 | 4.3 | 9.3 | 7.6 |
| 28 | | 300 | 10 | 1.8 | 8.2 | 7.1 |
| 29 | | 320 | 10 | 3.0 | 9.0 | 7.8 |
| 30 | | 320 | 20 | 3.9 | 9.8 | 8.2 |
| 31 | Fe-Co-Mn-Na 100:2:10:2 | 260 | 10 | 4.9 | 6.8 | 5.3 |
| 32 | | 280 | 10 | 3.2 | 8.6 | 6.8 |
| 33 | | 300 | 10 | 2.1 | 9.0 | 7.3 |
| 34 | | 320 | 10 | 3.1 | 10.0 | 7.7 |
| 35 | | 320 | 19 | 3.8 | 10.3 | 7.9 |
| 36 | Fe-Co-Mn-Na 100:5:10:2 | 260 | 10 | 4.3 | 6.2 | 4.8 |
| 37 | | 280 | 10 | 3.4 | 8.1 | 6.1 |
| 38 | | 300 | 10 | 2.3 | 8.9 | 6.9 |
| 39 | | 320 | 10 | 2.3 | 9.3 | 7.0 |
| 40 | | 320 | 20 | 2.7 | 9.2 | 6.8 |
| 41 | | 320 | 30 | 2.7 | 9.0 | 6.7 |
| 42 | | 320 | 40 | 2.8 | 8.9 | 6.5 |
| 43 | | 320 | 47 | 2.7 | 8.7 | 6.5 |
| 44 | | 320 | 48 | 2.7 | 8.7 | 6.4 |
| 45 | Fe-Co-Mn-Na 100:8:10:2 | 260 | 10 | 4.3 | 6.3 | 4.8 |
| 46 | | 280 | 10 | 5.0 | 10.1 | 7.5 |
| 47 | | 300 | 10 | 4.0 | 10.7 | 7.8 |
| 48 | | 320 | 10 | 3.9 | 10.8 | 7.7 |
| 49 | | 340 | 10 | 3.8 | 10.9 | 7.8 |

| Example | Catalyst | T/°C | Time(hours) | Olefin: paraffin molar ratio | | |
|---|---|---|---|---|---|---|
| | | | | C2 | C3 | C4 |
| 50 | | 360 | 10 | 0.2 | 2.4 | 2.0 |
| 51 | | 260 | 10 | 3.6 | 7.1 | 5.3 |
| 52 | FT-57 | 280 | 10 | 1.3 | 6.7 | 5.5 |
| 53 | Fe-Co-Mn-Na | 300 | 10 | 2.3 | 10.1 | 7.5 |
| 54 | 100:10:10:2 | 320 | 10 | 4.3 | 11.1 | 8.0 |
| 55 | | 320 | 20 | 4.9 | 10.7 | 7.6 |

Table 15 studies the effect various alkali metals on the CO hydrogenation. Iron based catalysts were prepared with a manganese and a cobalt promotor and the alkali metal varied between Na, K and Li. The catalysts were prepared using a method similar to that described above. The catalyst precursors of the catalysts studied in Table 15 were prepared as follows:

Examples 56-65: Iron powder, cobalt (II) nitrate hexahydrate, manganese (II) nitrate tetrahydrate, lithium carbonate were mixed together with molar ratio of Fe:Co:Mn:Li of 100:5:10:2, and the mixture was ground to uniformity, citric acid was added to the mixture and the mixture ground once more to uniformity, wherein the weight ratio of citric acid to iron powder was 1:1. The obtained mixtures were dried at 80°C for 24 hours. The dried mixtures (without calcination) were ground into powder.

Examples 66-70: Iron powder, cobalt (II) nitrate hexahydrate, manganese (II) nitrate tetrahydrate, sodium carbonate were mixed together with molar ratio of Fe:Co:Mn:Na of 100:5:10:2, and the mixture was ground to uniformity, citric acid was added to the mixture and the mixture ground once more to uniformity, wherein the weight ratio of citric acid to iron powder was 1:1. The obtained mixtures were dried at 80°C for 24 hours. The dried mixtures (without calcination) were ground into powder.

Examples 71-76: Iron powder, cobalt (II) nitrate hexahydrate, manganese (II) nitrate tetrahydrate, potassium carbonate were mixed together with molar ratio of Fe:Co:Mn:K of 100:5:10:2, and the mixture was ground to uniformity, citric acid was added to the mixture and the mixture ground once more to uniformity, wherein the weight ratio of citric acid to iron powder of 1:1. The obtained mixtures were dried at 80°C for 24 hours. The dried mixtures (without calcination) were ground into powder.

**Table 15**

| Example | Catalyst | T/°C | Time(hours) | Conversion % | | CO$_2$ Sel. % | Selectivity in hydrocarbons % | | | | |
|---|---|---|---|---|---|---|---|---|---|---|---|
| | | | | H$_2$ | CO | | CH$_4$ | C$_2$-C$_4^=$ | C$_2$-C$_4^0$ | C$_5^+$+C$_2$-$_4^=$ | C$_5^+$ |
| 56 | | 300 | 10 | 11.2 | 5.8 | 36.5 | 39.9 | 41.3 | 10.3 | 49.7 | 8.4 |
| 57 | | 300 | 20 | 22.1 | 14.7 | 33.7 | 26.9 | 28.3 | 8.2 | 64.9 | 36.6 |
| 58 | | 300 | 30 | 27.8 | 21.5 | 36.0 | 27.1 | 26.6 | 9.1 | 63.9 | 37.2 |
| 59 | | 300 | 40 | 28.9 | 23.8 | 38.2 | 28.4 | 26.0 | 9.7 | 62.0 | 36.0 |
| 60 | Fe-Co-Mn-Li 100:5:10:2 | 300 | 50 | 33.0 | 29.7 | 38.6 | 27.2 | 24.2 | 10.3 | 62.5 | 38.3 |
| 61 | | 300 | 60 | 38.9 | 35.8 | 39.7 | 26.4 | 23.7 | 11.2 | 62.4 | 38.7 |
| 62 | | 300 | 70 | 43.0 | 43.1 | 41.6 | 26.2 | 22.7 | 12.1 | 61.7 | 39.0 |
| 63 | | 300 | 80 | 42.5 | 42.9 | 42.7 | 27.4 | 22.0 | 12.9 | 59.7 | 37.7 |
| 64 | | 300 | 90 | 45.5 | 49.8 | 44.3 | 28.1 | 20.8 | 14.2 | 57.7 | 36.9 |
| 65 | | 300 | 94 | 47.5 | 52.4 | 44.7 | 28.6 | 20.0 | 14.6 | 56.8 | 36.8 |
| 66 | | 300 | 10 | 1.4 | 18.2 | 43.5 | 32.5 | 37.2 | 10.0 | 57.5 | 20.3 |
| 67 | Fe-Co-Mn-Na 100:5:10:2 | 300 | 20 | 16.8 | 34.2 | 45.3 | 30.9 | 31.0 | 11.4 | 57.7 | 26.7 |
| 68 | | 300 | 30 | 21.9 | 43.3 | 45.0 | 28.9 | 27.9 | 11.8 | 59.3 | 31.4 |
| 69 | | 300 | 40 | 29.2 | 53.2 | 45.1 | 25.7 | 25.6 | 12.0 | 62.3 | 36.7 |
| 70 | | 300 | 50 | 31.0 | 58.3 | 45.1 | 23.9 | 24.7 | 12.2 | 63.9 | 39.2 |
| 71 | | 300 | 10 | 13.7 | 40.4 | 65.6 | 33.7 | 42.0 | 9.3 | 57.0 | 15.0 |
| 72 | | 300 | 20 | 13.4 | 40.2 | 67.6 | 37.6 | 45.1 | 10.1 | 52.3 | 7.2 |
| 73 | Fe-Co-Mn-K 100:5:10:2 | 300 | 30 | 11.3 | 41.8 | 66.3 | 36.3 | 42.8 | 9.8 | 53.9 | 11.0 |
| 74 | | 300 | 40 | 9.1 | 44.1 | 65.8 | 35.4 | 42.2 | 9.7 | 54.9 | 12.7 |
| 75 | | 300 | 47 | 11.1 | 43.7 | 66.5 | 35.5 | 43.2 | 9.9 | 54.6 | 11.4 |
| 76 | | 300 | 70 | 12.8 | 41.2 | 67.6 | 35.5 | 46.5 | 10.4 | 54.1 | 7.6 |

Table 16 provides the molar ratio of olefins: paraffins for the C$_2$-C$_4$ hydrocarbons produced.

**Table 16**

| Example | Catalyst | T/ °C | Reaction time hours | Olefin: paraffin molar ratio | | |
|---|---|---|---|---|---|---|
| | | | | C$_2$ | C$_3$ | C$_4$ |
| 56 | | 300 | 10 | 2.33 | 7.27 | 5.43 |
| 57 | | 300 | 20 | 1.65 | 7.59 | 5.77 |
| 58 | | 300 | 30 | 1.21 | 7.25 | 5.57 |
| 59 | | 300 | 40 | 1.01 | 7.15 | 5.46 |
| 60 | Fe-Co-Mn-Li 100:5:10:2 | 300 | 50 | 0.80 | 6.46 | 4.96 |
| 61 | | 300 | 60 | 0.66 | 6.01 | 4.68 |
| 62 | | 300 | 70 | 0.50 | 5.60 | 4.41 |
| 63 | | 300 | 80 | 0.41 | 5.19 | 4.03 |
| 64 | | 300 | 90 | 0.29 | 4.39 | 3.36 |
| 65 | | 300 | 94 | 0.25 | 4.08 | 3.10 |

(continued)

| Example | Catalyst | T/ °C | Reaction time hours | Olefin: paraffin molar ratio | | |
|---|---|---|---|---|---|---|
| | | | | $C_2$ | $C_3$ | $C_4$ |
| 66 | Fe-Co-Mn-Na 100:5:10:2 | 300 | 10 | 2.03 | 7.44 | 5.83 |
| 67 | | 300 | 20 | 1.13 | 7.03 | 5.48 |
| 68 | | 300 | 30 | 0.84 | 6.60 | 5.21 |
| 69 | | 300 | 40 | 0.68 | 6.31 | 5.03 |
| 70 | | 300 | 50 | 0.59 | 6.20 | 5.00 |
| 71 | Fe-Co-Mn-K 100:5:10:2 | 300 | 10 | 2.93 | 7.23 | 5.49 |
| 72 | | 300 | 20 | 2.83 | 7.26 | 5.39 |
| 73 | | 300 | 30 | 2.74 | 7.17 | 5.31 |
| 74 | | 300 | 40 | 2.69 | 7.12 | 5.21 |
| 75 | | 300 | 47 | 2.69 | 7.14 | 5.17 |
| 76 | | 300 | 70 | 2.81 | 7.11 | 5.09 |

Table 17 studies the effect of manganese loading on the CO hydrogenation. Iron based catalysts were prepared with a manganese and a cobalt promotor and sodium. The catalysts were prepared using a method similar to that described above. In particular, the catalyst precursors of the catalysts of Table 17 were prepared as follows:

Examples 76-80: Iron powder, cobalt (II) nitrate hexahydrate, manganese (II) nitrate tetrahydrate, sodium carbonate were mixed together with molar ratio of Fe:Co:Mn:Na of 100:5:10:2, and the mixture was ground to uniformity, citric acid was added to the mixture and the mixture ground once more to uniformity, wherein the weight ratio of citric acid to iron powder of 1:1. The obtained mixtures were dried at 80°C for 24 hours. The dried mixtures (without calcination) were ground into powder.

Examples 81-88: Iron powder, cobalt (II) nitrate hexahydrate, manganese (II) nitrate tetrahydrate, sodium carbonate were mixed together with molar ratio of Fe:Co:Mn:Na of 100:5:20:2, and the mixture was ground to uniformity, citric acid was added to the mixture and the mixture ground once more to uniformity, wherein the weight ratio of citric acid to iron powder of 1:1. The obtained mixtures were dried at 80°C for 24 hours. The dried mixtures (without calcination) were ground into catalyst powder.

# Table 17

| Example | Catalyst | T/ °C | Reaction time hours | Conversion % | | CO₂ Sel. % | Selectivity in hydrocarbons % | | | | |
|---|---|---|---|---|---|---|---|---|---|---|---|
| | | | | $H_2$ | CO | | $CH_4$ | $C_2\text{-}C_4^{=}$ | $C_2\text{-}C_4^{0}$ | $C_5^{+}+C_{2\text{-}4}^{=}$ | $C_5^{+}$ |
| 76 | Fe-Co-Mn-Na 100:5:10:2 | 300 | 10 | 1.4 | 18.2 | 43.5 | 32.5 | 37.2 | 10.0 | 57.5 | 20.3 |
| 77 | | 300 | 20 | 16.8 | 34.2 | 45.3 | 30.9 | 31.0 | 11.4 | 57.7 | 26.7 |
| 78 | | 300 | 30 | 21.9 | 43.3 | 45.0 | 28.9 | 27.9 | 11.8 | 59.3 | 31.4 |
| 79 | | 300 | 40 | 29.2 | 53.2 | 45.1 | 25.7 | 25.6 | 12.0 | 62.3 | 36.7 |
| 80 | | 300 | 50 | 31.0 | 58.3 | 45.1 | 23.9 | 24.7 | 12.2 | 63.9 | 39.2 |
| 81 | Fe-Co-Mn-Na 100:5:20:2 | 300 | 10 | 34.4 | 39.8 | 44.8 | 21.7 | 28.9 | 6.9 | 71.4 | 42.5 |
| 82 | | 300 | 20 | 41.4 | 55.4 | 46.7 | 19.3 | 25.6 | 7.2 | 73.5 | 47.9 |
| 83 | | 300 | 30 | 47.6 | 67.3 | 47.6 | 18.3 | 23.9 | 7.6 | 74.2 | 50.3 |
| 84 | | 300 | 40 | 49.3 | 69.8 | 48.0 | 16.8 | 23.3 | 7.0 | 76.3 | 53.0 |
| 85 | | 300 | 50 | 49.4 | 71.1 | 47.8 | 15.9 | 23.0 | 6.6 | 77.5 | 54.6 |
| 86 | | 300 | 60 | 46.6 | 67.8 | 47.9 | 14.8 | 23.2 | 5.8 | 79.5 | 56.3 |
| 87 | | 300 | 70 | 46.5 | 68.8 | 48.0 | 14.2 | 23.3 | 5.6 | 80.2 | 56.9 |
| 88 | | 300 | 73.5 | 47.5 | 70.2 | 47.9 | 14.0 | 23.0 | 5.5 | 80.5 | 57.6 |

Table 18 provides the molar ratio of olefins: paraffins for the $C_2$-$C_4$ hydrocarbons produced.

**Table 18**

| | Catalyst | T/ °C | Reaction time hours | Olefin: paraffin molar ratio | | |
|---|---|---|---|---|---|---|
| | | | | $C_2$ | $C_3$ | $C_4$ |
| 76 | Fe-Co-Mn-Na 100:5:10:2 | 300 | 10 | 2.03 | 7.44 | 5.83 |
| 77 | | 300 | 20 | 1.13 | 7.03 | 5.48 |
| 78 | | 300 | 30 | 0.84 | 6.60 | 5.21 |
| 79 | | 300 | 40 | 0.68 | 6.31 | 5.03 |
| 80 | | 300 | 50 | 0.59 | 6.20 | 5.00 |
| 81 | Fe-Co-Mn-Na 100:5:20:2 | 300 | 10 | 2.30 | 8.63 | 6.84 |
| 82 | | 300 | 20 | 1.61 | 8.39 | 6.77 |
| 83 | | 300 | 30 | 1.23 | 7.96 | 6.36 |
| 84 | | 300 | 40 | 1.30 | 7.91 | 6.19 |
| 85 | | 300 | 50 | 1.39 | 7.80 | 6.05 |
| 86 | | 300 | 60 | 1.78 | 8.11 | 6.18 |
| 87 | | 300 | 70 | 1.92 | 8.15 | 6.17 |
| 88 | | 300 | 73.5 | 1.91 | 8.12 | 6.15 |

Table 19 studies the effect of feedstock composition on the CO hydrogenation. Iron based catalysts were prepared with a manganese and a cobalt promotor and sodium. The catalysts were prepared using a method similar to that described above. The reaction was performed using syngas with varying ratios of $H_2$:CO.

The catalyst precursors of the catalysts of Table 19 were prepared as follows:

Examples 89-110: Iron powder, cobalt (II) nitrate hexahydrate, manganese (II) nitrate tetrahydrate, sodium carbonate were mixed together with molar ratio of Fe:Co:Mn:Na of 100:5:10:2, and the mixture was ground to uniformity, citric acid was added to the mixture and the mixture ground once more to uniformity, wherein the weight ratio of citric acid to iron powder of 4:1. The obtained mixtures were dried at 80°C for 24 hours. The dried mixtures (without calcination) were ground into powder.

## Table 19

| Example | Catalyst | T/ °C | Time (hours) | Conversion % | | CO$_2$ Sel. % | Selectivity in hydrocarbons % | | | | |
|---|---|---|---|---|---|---|---|---|---|---|---|
| | | | | H$_2$ | CO | | CH$_4$ | C$_2$-C$_4^=$ | C$_2$-C$_4^0$ | C$_5^+$+C$_2$-$_4^=$ | C$_5^+$ |
| 89 | | 260 | 10 | 5.0 | 5.6 | 39.2 | 10.9 | 15.9 | 3.2 | 86.0 | 70.1 |
| 90 | Fe-Co-Mn-Na | 280 | 10 | 33.6 | 35.6 | 39.9 | 8.2 | 18.3 | 3.4 | 88.5 | 70.2 |
| 91 | Na | 300 | 10 | 39.3 | 57.0 | 47.0 | 11.4 | 21.2 | 4.5 | 84.2 | 62.9 |
| 92 | 100:5:10:2 | 320 | 10 | 43.7 | 64.3 | 48.2 | 16.2 | 24.3 | 5.1 | 78.7 | 54.5 |
| 93 | Molar ratio | 320 | 20 | 40.3 | 58.6 | 48.2 | 16.6 | 24.3 | 4.8 | 78.7 | 54.4 |
| 94 | of | 320 | 30 | 40.0 | 59.4 | 48.3 | 16.9 | 24.3 | 4.8 | 78.3 | 54.0 |
| 95 | H$_2$:CO of | 320 | 40 | 41.0 | 60.6 | 48.1 | 17.0 | 24.2 | 4.7 | 78.3 | 54.1 |
| 96 | 1:1 | 320 | 47 | 42.5 | 62.9 | 48.0 | 17.1 | 24.2 | 4.8 | 78.1 | 54.0 |
| 97 | | 320 | 48 | 43.0 | 63.1 | 48.2 | 17.2 | 24.3 | 4.9 | 78.0 | 53.7 |
| 98 | | 300 | 10 | 28.5 | 97.5 | 26.2 | 10.5 | 11.0 | 3.3 | 86.2 | 75.2 |
| 99 | | 300 | 20 | 29.2 | 98.1 | 26.1 | 9.8 | 11.7 | 3.7 | 86.6 | 74.9 |
| 100 | | 300 | 30 | 28.9 | 98.2 | 25.8 | 9.5 | 11.9 | 3.8 | 86.7 | 74.8 |
| 101 | Fe-Co-Mn-Na | 300 | 40 | 27.6 | 97.9 | 26.5 | 9.1 | 11.9 | 3.7 | 87.3 | 75.4 |
| 102 | Na | 300 | 50 | 28.1 | 98.1 | 26.2 | 9.0 | 12.0 | 3.7 | 87.3 | 75.3 |
| 103 | 100:5:10:2 | 300 | 60 | 28.2 | 98.2 | 26.1 | 8.9 | 12.0 | 3.7 | 87.4 | 75.3 |
| 104 | Molar ratio of | 300 | 70 | 28.0 | 98.2 | 26.1 | 8.9 | 12.2 | 3.7 | 87.4 | 75.2 |
| 105 | H$_2$:CO of | 300 | 80 | 30.3 | 98.4 | 25.6 | 8.8 | 12.1 | 3.8 | 87.5 | 75.4 |
| 106 | 2:1 | 300 | 87 | 28.9 | 98.2 | 25.9 | 8.5 | 12.0 | 3.7 | 87.8 | 75.8 |
| 107 | | 300 | 100 | 30.1 | 98.3 | 25.6 | 8.7 | 12.3 | 3.8 | 87.5 | 75.2 |
| 108 | | 300 | 110 | 29.5 | 98.3 | 25.7 | 8.6 | 12.3 | 3.7 | 87.8 | 75.5 |
| 109 | | 300 | 120 | 29.5 | 98.2 | 25.5 | 8.4 | 12.4 | 3.7 | 87.9 | 75.6 |

| Example | Catalyst | T/ °C | Time (hours) | Conversion % | | CO$_2$ Sel. % | Selectivity in hydrocarbons % | | | | |
|---|---|---|---|---|---|---|---|---|---|---|---|
| | | | | H$_2$ | CO | | CH$_4$ | C$_2$-C$_4^=$ | C$_2$-C$_4^0$ | C$_5^+$+C$_2$-$_4^=$ | C$_5^+$ |
| 110 | | 300 | 124 | 30.7 | 98.0 | 25.1 | 8.5 | 12.4 | 3.7 | 87.8 | 75.4 |

Table 20 provides the molar ratio of olefins: paraffins for the C$_2$-C$_4$ hydrocarbons produced.

**Table 20**

| Example | Catalyst | T/ °C | Reaction time hours | Olefin: paraffin molar ratio | | |
|---|---|---|---|---|---|---|
| | | | | $C_2$ | $C_3$ | $C_4$ |
| 89 | | 260 | 10 | 4.25 | 6.15 | 4.79 |
| 90 | Fe-Co-Mn-Na 100:5:10:2 Molar ratio of $H_2$:CO of 1:1 | 280 | 10 | 3.43 | 8.14 | 6.09 |
| 91 | | 300 | 10 | 2.33 | 8.92 | 6.87 |
| 92 | | 320 | 10 | 2.28 | 9.25 | 7.00 |
| 93 | | 320 | 20 | 2.68 | 9.18 | 6.81 |
| 94 | | 320 | 30 | 2.74 | 8.96 | 6.65 |
| 95 | | 320 | 40 | 2.77 | 8.85 | 6.52 |
| 96 | | 320 | 47 | 2.69 | 8.74 | 6.46 |
| 97 | | 320 | 48 | 2.68 | 8.71 | 6.44 |
| 98 | Fe-Co-Mn-Na 100:5:10:2 Molar ratio of $H_2$:CO of 2:1 | 300 | 10 | 1.51 | 6.64 | 5.75 |
| 99 | | 300 | 20 | 1.32 | 6.96 | 5.90 |
| 100 | | 300 | 30 | 1.26 | 7.01 | 5.85 |
| 101 | | 300 | 40 | 1.34 | 7.13 | 5.94 |
| 102 | | 300 | 50 | 1.31 | 7.13 | 5.93 |
| 103 | | 300 | 60 | 1.30 | 7.14 | 5.95 |
| 104 | | 300 | 70 | 1.32 | 7.21 | 5.98 |
| 105 | | 300 | 80 | 1.29 | 7.17 | 5.96 |
| 106 | | 300 | 87 | 1.35 | 7.25 | 5.64 |
| 107 | | 300 | 100 | 1.31 | 7.23 | 5.99 |
| 108 | | 300 | 110 | 1.35 | 7.27 | 6.01 |
| 109 | | 300 | 120 | 1.37 | 7.31 | 6.06 |
| 110 | | 300 | 124 | 1.34 | 7.26 | 6.02 |

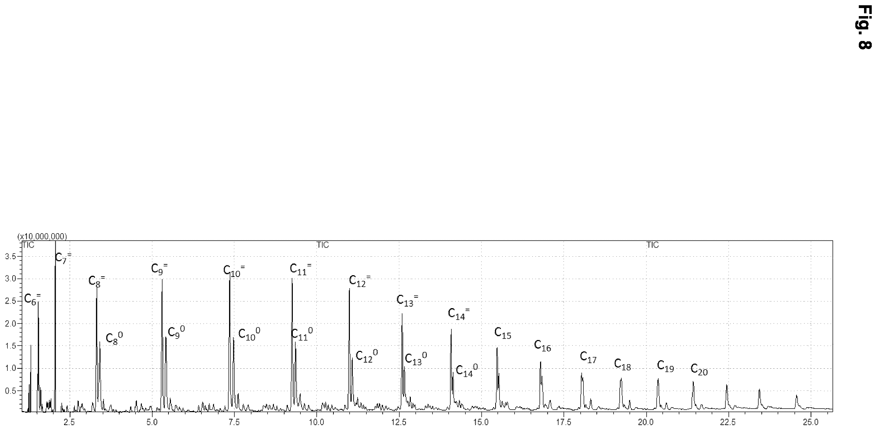

Tables 21 and 22 study CO hydrogenation under a variety of conditions using a Fe-Co-Mn-Na catalyst (100:5:20:2). The GC-MS spectrum showing the product profile of example 111 is shown in Figure 8.

**Table 21**

| Example | T/°C | P/MPa | $H_2$/CO | Time/hrs | conversion /% | | $CO_2$ sel. /% | Selectivity in hydrocarbons /(%) | | | |
|---|---|---|---|---|---|---|---|---|---|---|---|
| | | | | | CO | $H_2$ | | $CH_4$ | $C_{2-4}^=$ | $C_{2-4}^0$ | $C_{5+}$ |
| 111 | 300 | 1.0 | 1 | 72 | 70.50 | 47.85 | 47.21 | 13.57 | 22.31 | 5.35 | 58.76 |
| 112 | 280 | 1.0 | 2 | 300 | 87.23 | 20.43 | 24.61 | 12.39 | 9.77 | 6.66 | 61.64 |
| 113 | 300 | 1.0 | 2 | 160 | 93.54 | 19.39 | 27.24 | 10.72 | 7.86 | 8.56 | 64.58 |

Table 22

| Example | T/°C | P/MPa | H₂/CO | Time/hrs | Selectivity in liquid products/(%) | | | | | |
|---|---|---|---|---|---|---|---|---|---|---|
| | | | | | $C_{5-11}$ | $C_{5-11}^{=}$ | $C_{5-11}^{0}$ | $C_{12+}$ | $C_{12+}^{=}$ | $C_{12+}^{0}$ |
| 111 | 300 | 1.0 | 1 | 72 | 52.82 | 38.01 | 14.81 | 47.18 | 23.93 | 23.25 |
| 112 | 280 | 1.0 | 2 | 300 | 53.78 | 16.63 | 37.15 | 46.22 | - | - |
| 113 | 300 | 1.0 | 2 | 160 | 57.38 | 22.42 | 34.96 | 42.62 | - | - |

Iron powder has applied as iron source with a complexing agent to prepare catalysts. The preparation does not require calcination thus saving energy and reducing emissions. The prepared catalysts show high CO conversion, low $CH_4$ selectivity, high olefin selectivity, and stability. The addition of alkali metal and optionally transition metals (e.g. Co, Mn) promoted the olefin selectivity, both in gaseous and liquid products. The catalysts can also be used for the production of fuels (gasoline, diesel, aviation/jet fuel) at a higher $H_2$:CO molar ratio in feedstock.

The catalysts prepared for $CO_2$ hydrogenation also can be applied in the CO hydrogenation and vice versa.

## 3. $CO_2$ Hydrogenation to Jet fuel

Jet fuel or aviation fuel are used in gas-turbine engines to power aircraft. The main components of jet fuel are linear and branched alkanes and cycloalkanes with a typical carbon chain-length distribution of $C_8$-$C_{18}$, and preferably with a carbon chain length distribution of $C_8$-$C_{16}$.

The generation of the jet fuel range hydrocarbons in the product of $CO_2$ hydrogenation using catalysts disclosed herein.

### Catalyst preparation

Catalysts were prepared by the organic combustion method. A Fe-Mn-K catalyst precursor was prepared by mixing citric acid monohydrate (99%, Sigma-Aldrich) with iron (III) nitrate nonahydrate(98%, Sigma-Aldrich), manganese(II) nitrate tetrahydrate (97%, Sigma-Aldrich) and potassium nitrate (99%, Sigma-Aldrich). The molar ratio of citric acid: (Fe+Mn+K) was 2, and weight ratio of (Fe- and Mn- and K-precursors + citric acid):water was 2:1. The mixture was stirred to form a homogeneous aqueous solution, and heated at 50°C for 1 to 2 hours to obtain a citric acid-based slurry. This paste is ignited at 350 °C (furnace temperature) in static air for 4 hours to produce a powder.

Catalysts with different transition metal (Mn, Cu, Zn) promoters were also prepared using the same method, the catalysts of Fe-Cu-K and Fe-Zn-K were prepared with transition metal precursors of copper (II) nitrate trihydrate (99-104%, Sigma-Aldrich), and zinc nitrate hexahydrate (98%, Sigma-Aldrich) respectively.

Catalysts with different base metal promoters of Fe-Mn-Li, Fe-Mn-Na, and Fe-Mn-Cs were prepared with precursors of lithium carbonate (99%, Sigma-Aldrich), sodium carbonate (99.6%, Acros Organics), cesium carbonate (99%, Sigma-Aldrich) respectively.

In each case, the molar ratio of Fe: transition metal: base metal was 10:1:1.

Fe-Mn-K catalysts were also prepared using organic compounds other than citric acid, the organic compounds used as urea (Bio-Reagent, Sigma-Aldrich), tannic acid (ACS reagent, Sigma-Aldrich), Ethylenediamine Tetraacetic Acid (EDTA, 99.5%, Fisher Scientific), oxalic acid (99.0%, Sigma-Aldrich), Nitrilotriacetic acid (NTA, 99%, Sigma-Aldrich), Diethylenetriaminepentaacetic acid (DTPA, 98%, Sigma-Aldrich), tartaric acid (99.5%, Sigma-Aldrich), N-(2-Hydroxyethyl) ethylenediamine-N,N',N'- triacetic acid (HEDTA,98%, Sigma-Aldrich), salicylic acid (99.0%, Sigma-Aldrich). The catalysts were prepared with citric acid as the organic compound unless otherwise stated.

### Catalysts performance evaluation.

$CO_2$ hydrogenation experiments were carried out in a fixed bed reactor as previously described. Prior to the reaction, the catalyst precursor was *in situ* reduced with syngas ($H_2$:CO=2:1) at atmospheric pressure, with a GHSV (gas hourly space velocity) of 1000 mL g$^{-1}$ hr$^{-1}$, at 320 °C for 24 hours. After the reactor temperature cooling down to below 50 °C, and the mixture of gas with an $H_2$/$CO_2$ ratio of 3 and $N_2$ (as an internal standard gas) was introduced into the reactor, the gas flow of 40 mL min$^{-1}$ (GSVH =2 400 mL g$^{-1}$ hr$^{-1}$). The reactor was heated with a heating rate of 2 °C/min until the reaction temperature (300 °C). The reaction pressure was fixed at 10 bar (1 MPa) by a back pressure regulator.

The effluent gaseous products were analysed on an online Gas Chromatograph (Perkin Elmer Clarus 580 GC) with flame ionization detector (FID) and thermal conductivity detector (TCD) detectors, the collected liquid products were analysed by Gas Chromatograph Mass Spectrometer (SHIMADZU GCMS-QP2010 SE).

The $CO_2$ and $H_2$ conversion, products selectivity were calculated as previously described.

**Characterisation Methods**

The powder X-ray diffraction (XRD) analyses of catalysts used a Cu K$\alpha$ (0.15418 nm) X-ray source (25 kV, 40 mA) on a Bruker D8 Advance diffractometer. Diffraction patterns were recorded over a 10-80° 2$\theta$ angular range using a step size of 0.016 °. Crystallite sizes were determined using the Scherrer equation.

X-Ray Photoelectron Spectroscopy (XPS) of samples was performed using a Thermo Fisher Scientific Nexsa spectrometer. Samples were analysed using a micro-focused monochromatic Al X-ray source (72 W) over an area of approximately 400 mm. Data were recorded at pass energies of 150 eV for survey scans and 40 eV for high resolution scan with 1 eV and 0.1 eV step sizes respectively. Charge neutralisation was achieved using a combination of low energy electrons and argon ions. The resulting spectra were analysed using Casa XPS peak fitting software and sample charging corrected using the C 1s signal at 284.8 eV as reference.

The morphology of the catalysts was characterised by scanning electron microscopy (SEM) on a scanning electron microscope (SEM, JEOL 840F).

High-resolution transmission electron microscopy (HRTEM) images were obtained in a probe corrected JEOL ARM200F operated at 200 kV with a Gatan GIF Quantum 965 ER spectrometer.

**Catalytic performance of Fe-Mn-K (10:1:1) catalyst on $CO_2$ hydrogenation.**

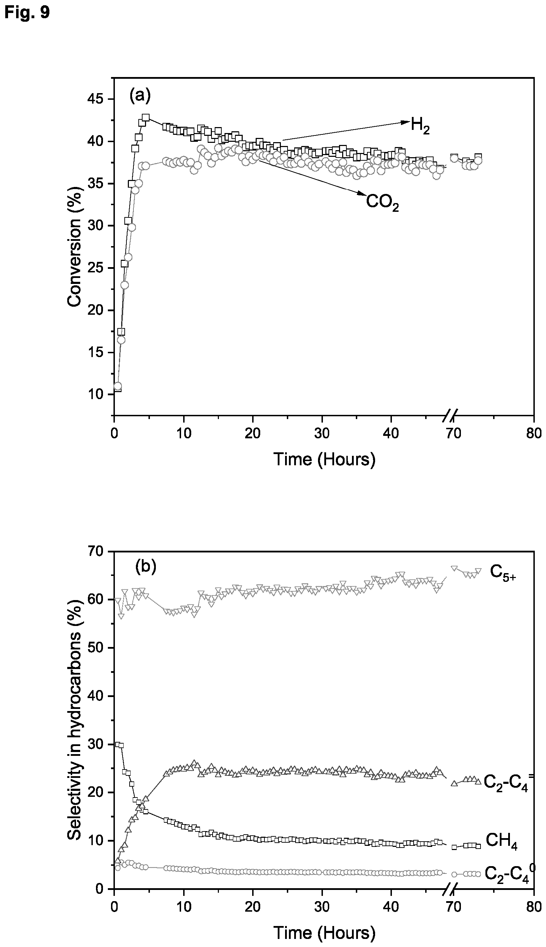

The conversion of $CO_2$ and $H_2$ with the Fe-Mn-K (10:1:1) catalyst prepared with citric acid as described above in illustrated in Figure 9 in terms of product selectivity. Figure 9 shows that the $CO_2$ and $H_2$ conversion increases rapidly with the reaction time in the first 5 hours, and reached around 40%; the methane selectivity decreased from 30% to 10% from the beginning of reaction until a reaction time of 20 hours. In contrast, the liquid products ($C_{5+}$) selectivity kept stable at around 60% and showed a slight increase with the reaction time.

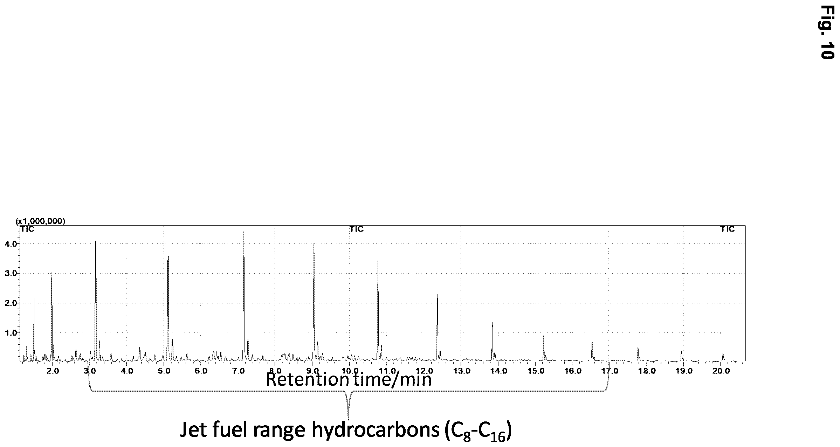

The GC-MS spectrum of collected liquid products from the $CO_2$ hydrogenation is presented in Figure 10. Figure 10 shows that the Fe-Mn-K catalyst had a high selectivity of jet fuel range hydrocarbons in liquid products, and the total jet fuel range hydrocarbons selectivity reached 47.8%.

**Catalyst Characterisation**

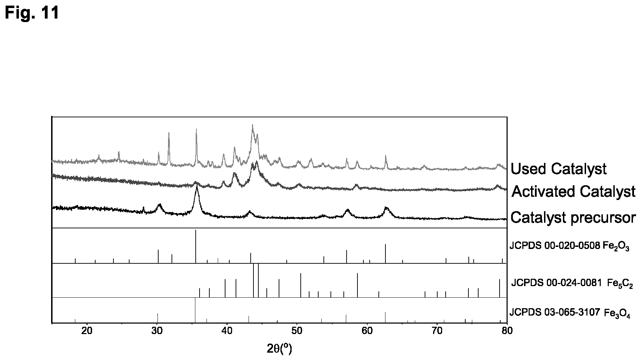

The powder X-ray diffraction (XRD) spectrum of above catalyst precursor, activated catalyst and the used catalyst is presented in Figure 11.

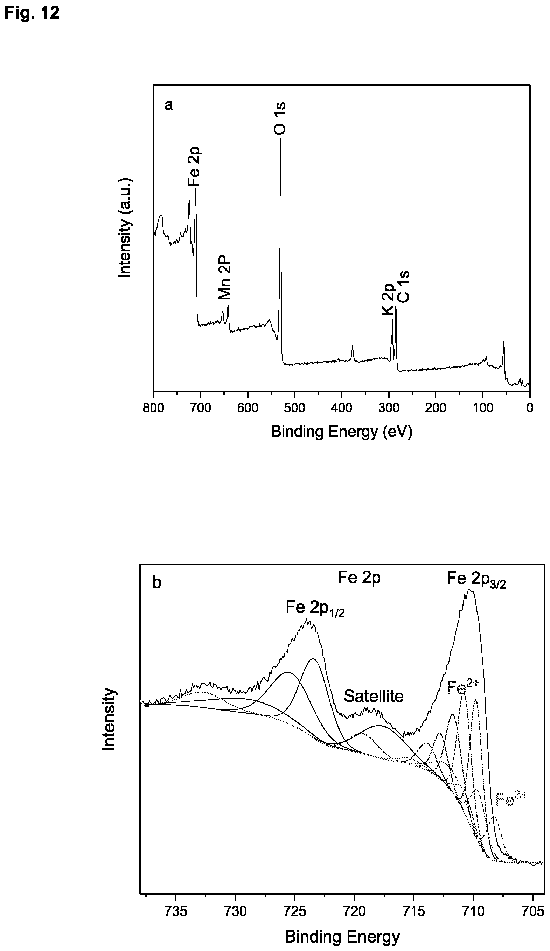

The surface elemental compositions and oxidation states of the metals were analysed by using XPS in the region of 0-1350 eV. The survey spectrum (Figure 12a) indicated that the sample contains Fe, Mn, K, and O. Figure 12b showed the XPS spectrum of Fe 2p region, which can be fitted with two spin-orbit doublets of Fe $2p_{3/2}$ and Fe $2p_{1/2}$ peaks with a binding energy gap of 13.7 eV and a shakeup satellite which assigned to $Fe^{3+}$, the peaks are consistent with reported of $Fe_3O_4$. The molar ratio of $Fe^{2+}$: $Fe^{3+}$ of 1:2.34, that is very close to the stoichiometry of $Fe_3O_4$.

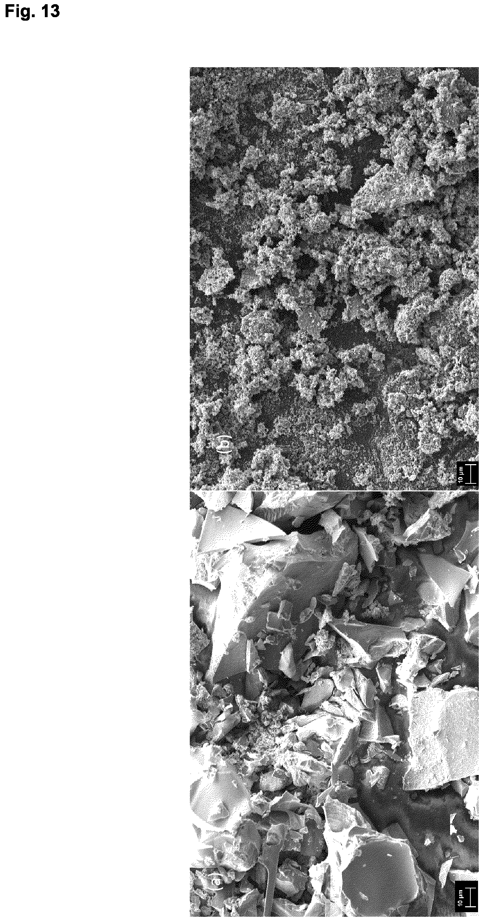

The scanning electron microscopy (SEM) images of the catalyst and used catalysts were shown in Figure 13. The catalyst precursor showed clearly packed, regular particles (Figure 13(a)), and obvious changes take place in the morphology of the catalyst after use (Figure 13(b)) indicating changes of the surface of the catalyst before and after reactions.

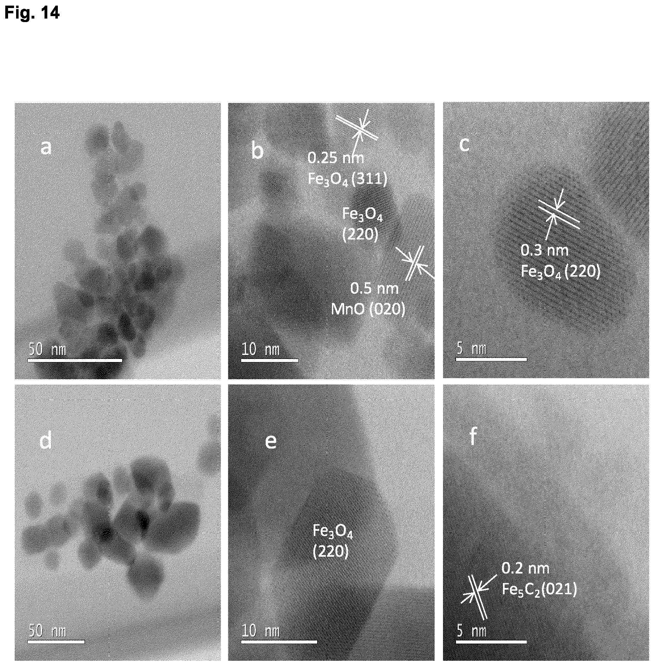

The high-resolution transmission electron microscopy (HRTEM) of the catalyst precursor and used catalyst is shown in Figure 14. Fig. 14a shows the particle size of the catalyst precursor (approx. 15nm) and there is no obvious change in particle size after reaction (Fig. 14d). The lattice spaces of 0.25 and 0.3 nm correspond respectively to the (311) and (220) planes of $Fe_3O_4$ on catalyst precursor (Fig. 14b and Fig. 14c). Besides the $Fe_3O_4$ phase (Fig. 14e), an $Fe_5C_2$ phase on used catalysts was observed (Fig. 14f).

**Effect of transition metal on product profile**

Catalysts of Fe-Zn-K and Fe-Cu-K were prepared with the same method as catalyst Fe-Mn-K. The catalytic performances of $CO_2$ hydrogenation on different catalysts were shown in Table 23. The molar ratio of K and Mn(Zn or Cu) to Fe was 1:10, data were obtained at the reaction time of 20 hours.

**Table 23**

| Catalyst | Conversion (%) | | CO selectivity (%) | Selectivity in hydrocarbons (%) | | | | |
|---|---|---|---|---|---|---|---|---|
| | $H_2$ | $CO_2$ | | $CH_4$ | $C_{2-4}=$ | $C_{2-4}0$ | $C_{5+}$ | $C_{8-16}$ |
| Fe | 48.6 | 40.5 | 6.6 | 32.2 | 15.1 | 15.5 | 37.3 | - |
| Fe-Zn-K | 36.0 | 35.5 | 9.1 | 10.5 | 24.2 | 3.6 | 61.7 | 45.1 |
| Fe-Cu-K | 39.4 | 38.2 | 8.6 | 8.3 | 18.5 | 4.8 | 68.3 | 40.8 |
| Fe-Mn-K | 39.5 | 38.2 | 5.6 | 10.4 | 24.2 | 3.5 | 61.9 | 47.8 |
| $C_{2-4}=$: $C_2$-$C_4$ olefin, $C_{2-4}0$: $C_2$-$C_4$ paraffin; $C_{5+}$: liquid products; $C_{8-16}$: Jet fuel range hydrocarbons. | | | | | | | | |

**Effects of base metals on product profile**

The different base metals were also applied as promoters on the catalysts for the $CO_2$ hydrogenation, the catalytic performances are listed in Table 24. The molar ratio of base metal and Mn to Fe was 1:10, and data was obtained at the reaction time of 20 hours

**Table 24**

| Catalyst | Conversion (%) | | CO selectivity (%) | Selectivity in hydrocarbons (%) | | | | |
|---|---|---|---|---|---|---|---|---|
| | $H_2$ | $CO_2$ | | $CH_4$ | $C_{2-4}=$ | $C_{2-4}0$ | $C_{5+}$ | $C_{8-16}$ |
| Fe-Mn | 34.2 | 29.7 | 10.6 | 43.0 | 18.2 | 10.8 | 28.0 | - |
| Fe-Mn-Li | 38.3 | 32.9 | 8.5 | 34.6 | 14.3 | 17.0 | 34.1 | - |
| Fe-Mn-Na | 38.4 | 35.2 | 6.5 | 13.4 | 25.8 | 4.9 | 55.9 | 44.4 |
| Fe-Mn-K | 39.5 | 38.2 | 5.6 | 10.4 | 24.2 | 3.5 | 61.9 | 47.8 |
| Fe-Mn-Cs | 36.0 | 33.7 | 9.2 | 12.0 | 23.8 | 3.8 | 60.4 | 44.0 |

It can be seen from Table 24, the Na, K, and Cs have shown both high activities on $CO_2$ hydrogenation and high selectivity on jet fuel range, The Fe-Mn-K catalyst showed slightly better performance on $CO_2$ conversion and target product selectivity compared with the catalysts of Fe-Mn-Na and Fe-Mn-Cs.

**Effects of organic compound on product profile**

A series of catalysts of Fe-Mn-K (molar ratio 10:1:1) have been prepared with different organic compounds applied in catalysts preparation, their catalytic performance on $CO_2$ hydrogenation are presented in Table 25.

**Table 25**

| Organic compound | Conversion (%) | | CO selectivity (%) | Selectivity in hydrocarbons (%) | | | | |
|---|---|---|---|---|---|---|---|---|
| | $H_2$ | $CO_2$ | | $CH_4$ | $C_{2-4}=$ | $C_{2-4}0$ | $C_{5+}$ | $C_{8-16}$ |
| None | 27.0 | 28.6 | 6.5 | 14.1 | 26.0 | 5.2 | 54.7 | - |
| Urea | 34.4 | 35.0 | 5.8 | 14.3 | 27.6 | 4.6 | 53.5 | 38.3 |
| Tannic acid | 39.2 | 38.8 | 5.0 | 16.2 | 26.4 | 4.8 | 52.6 | 34.5 |
| EDTA | 39.6 | 40.6 | 7.0 | 13.5 | 21.6 | 4.4 | 60.5 | 51.0 |
| Citric acid | 39.5 | 38.2 | 5.6 | 10.4 | 24.2 | 3.5 | 61.9 | 47.8 |
| Oxalic acid | 36.7 | 37.0 | 7.4 | 9.8 | 25.4 | 3.5 | 61.3 | 47.9 |
| NTA | 39.5 | 42.5 | 5.1 | 7.2 | 18.3 | 2.6 | 71.9 | 49.0 |
| DTPA | 42.0 | 44.0 | 6.2 | 9.6 | 19.0 | 3.4 | 68.0 | 53.3 |
| Tartaric acid | 40.1 | 41.8 | 4.6 | 7.9 | 18.9 | 2.7 | 70.4 | 50.2 |
| HEDTA | 42.3 | 41.5 | 4.9 | 9.6 | 20.2 | 3.1 | 67.0 | 47.0 |

(continued)

| Organic compound | Conversion (%) | | CO selectivity (%) | Selectivity in hydrocarbons (%) | | | | |
|---|---|---|---|---|---|---|---|---|
| | $H_2$ | $CO_2$ | | $CH_4$ | $C_{2-4}=$ | $C_{2-4}0$ | $C_{5+}$ | $C_{8-16}$ |
| Salicylic acid | 37.8 | 37.3 | 7.2 | 12.6 | 22.0 | 3.8 | 61.5 | 49.5 |
| Sugar | 36.5 | 37.4 | 8.8 | 10.3 | 21.5 | 3.9 | 64.3 | 45.8 |
| Flour powder | 36.5 | 35.6 | 7.2 | 12.1 | 25.9 | 4.0 | 58.0 | 39.8 |

It is clear that, compared with the catalyst prepared without organic compound, all the Fe-Mn-K catalysts prepared with organic compounds showed both higher $CO_2$ conversion and higher jet fuel range hydrocarbons selectivity, the catalysts prepared with EDTA, citric acid, oxalic acid, NTA, DTPA, Tartaric acid, HEDTA, and salicylic acid showed better catalytic performance.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise paragraphed. No language in the specification should be construed as indicating any non-paragraphed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the paragraphs appended hereto as permitted by applicable law.

## Claims

1. A catalyst for hydrogenation of carbon dioxide and/or carbon monoxide, wherein the catalyst comprising an iron species, manganese or an oxide thereof, cobalt or an oxide thereof, and an alkali metal.

2. A catalyst according to claim 1, comprising an iron carbide, manganese or an oxide thereof, cobalt or an oxide thereof, and an alkali metal.

3. A catalyst according to claim 1 or claim 2, wherein the alkali metal is potassium.

4. A catalyst according to any one of claims 1 to 3, wherein the molar ratio of iron: manganese is between about 100:1 to about 4:1.

5. A catalyst according to claim 4, wherein the molar ratio of iron:manganese is between about 15:1 to about 5:1.

6. A catalyst according to any one of claims 3 to 5, wherein the molar ratio of iron: potassium is about 100:1 to about 2:1.

7. A catalyst according to claim 6, wherein the molar ratio of iron: potassium is about 20:1 to about 4:1.

8. A catalyst according to any one of the preceding claims, wherein the iron carbide is $Fe_5C_2$.

9. A catalyst according to any one of the preceding claims, further comprising one or more of zinc, copper or a salt, oxide or hydroxide thereof.

10. A method for preparing a catalyst suitable for hydrogenation of carbon dioxide and/or carbon monoxide, comprising:

   (a) providing a catalyst precursor by (i) combining an iron species, a further transition metal selected from managanese, zinc, cobalt and copper, or a salt, oxide or hydroxide thereof an alkali metal or salt thereof, a complexing agent and a solvent; (ii) agitating the mixture to provide a homogenous mixture; and (iii) heating the homogenous mixture of to partially remove the solvent and thereby provide a slurry or paste;

(b) subjecting said catalyst precursor to calcination; and

(c) activating said precursor.

11. A method according to claim 10, wherein the activation of step (c) is achieved by exposing the calcined precursor to a mixture of carbon monoxide and hydrogen gas at a temperature of about 250°C to about 500°C.

12. A method according to claim 10 or claim 11, wherein the complexing agent is selected from citric acid, tartaric acid, oxalic acid, EDTA (ethylenediaminetetraacetic acid), NTA (nitroilotiracetic acid), DTPA (diethylenetriaminepentaacetic acid), and HEDTA (N-(2-hydroxyethyl) ethylenediamine- N,N',N'-triacetic acid), or a salt thereof.

13. A method according to claim 12, wherein the complexing agent is citric acid.

14. A process for the hydrogenation of carbon dioxide comprising contacting a feedstock comprising hydrogen and carbon dioxide with a catalyst according to claims 1 to 9 at elevated temperature and pressure.

15. A process for the hydrogenation of carbon monoxide comprising contacting a feedstock comprising hydrogen and carbon monoxide with a catalyst according to claims 1 to 9 at elevated temperature and pressure.

16. A process for the production of hydrocarbons comprising contacting a feedstock comprising (i) hydrogen and (ii) carbon monoxide and/or carbon dioxide with a catalyst according to claims 1 to 9 at elevated temperature and pressure.

17. A process according to any one of claims 14 to 16, wherein the feedstock is contacted with the catalyst at a temperature of about 180°C to about 500°C and/or a pressure of about 500KPa to about 10 MPa.

18. A process according to claim 16 or claim 17, wherein the hydrocarbons are $C_5$+ hydrocarbons.

**Fig. 1**

Fixed bed reactor

Condenser

GC

GC-MS

CO+H$_2$+N$_2$

CO$_2$+H$_2$+N$_2$

N$_2$

**Fig. 2**

**Fig. 3**

**Fig. 4**

**Fig. 5**

**Fig. 6**

**Fig. 7**

**Fig. 8**

**Fig. 9**

**Fig. 10**

**Fig. 11**

**Fig. 12**

**Fig. 13**

**Fig. 14**